# EUROPEAN PATENT APPLICATION

(11) **EP 3 718 544 A1**
(43) Date of publication of application: **07.10.2020**
(21) Application number: 20160587.0
(22) Date of filing: 01.10.2014
(51) Int. Cl.: A61K 31/4155, A61K 31/4375, A61K 31/58, A61K 45/06, A61P 35/00, A61P 35/04, A61P 43/00

(54) **COMBINATION**

(30) Priority: 01.10.2013 US 201361885049 P; 30.01.2014 US 201461933379 P
(62) Divisional of application: 14790343.9
(71) Applicant: Novartis AG, 4056 Basel (CH)
(72) Inventor: CORNFELD, Mark J., Collegeville, PA Pennsylvania 19426 (US); KUMAR, Rakesh, Collegeville, PA Pennsylvania 19426 (US); MORRIS, Shannon Renae, Research Triangle Park, NC North Carolina 27709 (US)
(74) Representative: Ammelburg, Jan Moritz

(57) **Abstract**

A novel combination comprising a 17 α-hydroxylase/C17,20 lyase inhibitor, for example: (3β)-17-(pyridin-3-yl)androsta-5,16-dien-3-ol or a pharmaceutically acceptable salt or ester thereof, and an AKT inhibitor, for example: *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof, and optional additional antineoplastic agents; pharmaceutical compositions comprising the same and methods of using such combinations and compositions in the treatment of conditions in which the inhibition of 17 α-hydroxylase/C17,20 lyase and/or AKT inhibition is beneficial, e.g., cancer.

## Description

### FIELD OF INVENTION

The present invention relates to a method of treating cancer and to combinations useful in such treatment. In particular, the method relates to a novel combination comprising a 17 α-hydroxylase/C17,20 lyase (CYP17A1) inhibitor, suitably (3β)-17-(pyridin-3-yl)androsta-5,16-dien-3-ol or a pharmaceutically acceptable salt or ester thereof, with an AKT inhibitor, suitably: *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof, and optional additional antineoplastic agents; pharmaceutical compositions comprising the same and methods of using such combinations in the treatment of conditions in which the inhibition of CYP17A1 and/or AKT is beneficial, e.g., cancer.

### BACKGROUND OF THE INVENTION

Effective treatment of hyperproliferative disorders including cancer is a continuing goal in the oncology field. Generally, cancer results from the deregulation of the normal processes that control cell division, differentiation and apoptotic cell death and is characterized by the proliferation of malignant cells which have the potential for unlimited growth, local expansion and systemic metastasis. Deregulation of normal processes includes abnormalities in signal transduction pathways, and/or abnormalities in the regulation of gene transcription, and/or responses to factors (e.g., growth factors) which differ from those found in normal cells.

Many prostate cancers are characterized by dependence on the androgen receptor and genetic alterations in androgen receptor signaling. The primary mode of treatment for metastatic prostate cancer has historically focused on targeting androgen-androgen receptor signaling by decreasing the amount of ligand (androgens) available for binding to the androgen receptor.

Cytochrome P450 17A1, also known as 17 α-hydroxylase/C17,20 lyase (CYP17A1), is a key enzyme in the pathway that produces progestin, mineralocorticoid, glucocorticoid, androgen and estrogen. Inhibition of CYP17A1 provides a useful tool in targeting the androgen-AR signaling pathway; however, when this pathway is activated at the post-receptor ligand binding level or through nonhormonally mediated mechanisms, CYP17A1 inhibitors may not suffice. (Rini, B.I., and Small, E.J., Hormone-refractory prostate cancer Curr.Treat. Options Oncol. 2002;3:437; Singh, P., Yam, M., Russell, P.J., and Khatri, A., Molecular and traditional chemotherapy: a united front against prostate cancer. Cancer Lett. 2010;293:1).

Many prostate cancers are also characterized by constitutive, or otherwise abnormalactivation of the phosphoinositide 3-kinase (PI3K) signaling pathway. The PI3K pathway is among the most commonly activated in human cancer and the importance in carcinogenesis is well established (Samuels Y and Ericson K. Oncogenic PI3K and its role in cancer. Current Opinion in Oncology, 2006;18:77-82). Initiation of signaling begins with the phosphorylation of phosphatidylinositol-4, 5-bisphosphate (PIP2) to produce phosphatidylinositol-3, 4, 5-P3 (PIP3). PIP3 is a critical second messenger which recruits proteins that contain pleckstrin homology domains to the cell membrane where they are activated. The most studied of these proteins is AKT which promotes cell survival, growth, and proliferation. It has been shown that in many cases the mechanism of activation of PI3K signaling in prostate cancers is functional deficiencies of the tumor suppressor protein Pten.

Androgen deprivation therapy remains the standard of care for treatment of advanced prostate cancer. Despite an initial favorable response, almost all patients invariably progress to a more aggressive, castrate-resistant phenotype. Evidence indicates that the development of castrate-resistant prostate cancer is causally related to continue signaling of the androgen receptor.

Analysis of Akt levels in human tumors showed that Akt2 is overexpressed in a significant number of ovarian (J. Q. Cheung et al. Proc. Natl. Acad. Sci. U.S.A. 89:9267-9271(1992)) and pancreatic cancers (J. Q. Cheung et al. Proc. Natl. Acad. Sci. U.S.A. 93:3636-3641 (1996)). Similarly, Akt3 was found to be overexpressed in breast and prostate cancer cell lines (Nakatani et al. J. Biol.Chem. 274:21528-21532 (1999). It was demonstrated that Akt-2 was over-expressed in 12% of ovarian carcinomas and that amplification of Akt was especially frequent in 50% of undifferentiated tumors, suggesting that Akt may also be associated with tumor aggressiveness (Bellacosa, et al., Int. J. Cancer, 64, pp. 280-285, 1995). Increased Akt1 kinase activity has been reported in breast, ovarian and prostate cancers (Sun et al. Am. J. Pathol. 159: 431-7 (2001)).

The tumor suppressor PTEN, a protein and lipid phosphatase that specifically removes the 3' phosphate of Ptdlns(3,4,5)-P3, is a negative regulator of the PI3K/Akt pathway (Li et al. Science 275:1943-1947 (1997), Stambolic et al. Cell 95:29-39 (1998), Sun et al. Proc. Natl. Acad. Sci. U.S.A. 96:6199-6204 (1999)). Germline mutations of PTEN are responsible for human cancer syndromes such as Cowden disease (Liaw et al. Nature Genetics 16:64-67 (1997)). PTEN is deleted in a large percentage of human tumors and tumor cell lines without functional PTEN show elevated levels of activated Akt (Li et al. supra, Guldberg et al. Cancer Research 57:3660-3663 (1997), Risinger et al. Cancer Research 57:4736-4738 (1997)).

These observations demonstrate that the PI3K/Akt pathway plays important roles for regulating cell survival or apoptosis in tumorigenesis and/or cancer.

It would be useful to provide a novel therapy which provides more effective and/or enhanced treatment of an individual suffering the effects of cancer, suitably prostate cancer.

### SUMMARY OF THE INVENTION

One embodiment of this invention provides a combination comprising:
(i) a 17 α-hydroxylase/C17,20 lyase inhibiting compound; and
(ii) an AKT inhibiting compound; and
(iii) optional additional antineoplastic agents.

One embodiment of this invention provides a combination comprising:
(i) a 17 α-hydroxylase/C17,20 lyase inhibiting compound; and
(ii) a compound of Structure (II): 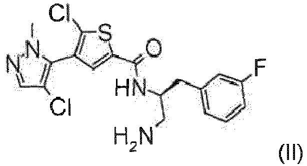 or a pharmaceutically acceptable salt thereof (collectively referred to herein as "Compound B"); and
(iii) optional additional antineoplastic agents.

One embodiment of this invention provides a combination comprising:
(i) a 17 α-hydroxylase/C17,20 lyase inhibitor of Structure (I): 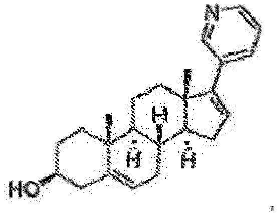 or a pharmaceutically acceptable salt or ester thereof (collectively referred to herein as "Compound A");
(ii) a compound of Structure (II): 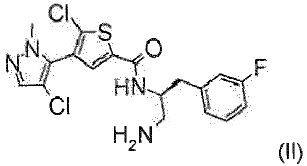 or a pharmaceutically acceptable salt thereof; and
(iii) optional additional antineoplastic agents.

One embodiment of this invention provides a combination comprising:
(i) a 17 α-hydroxylase/C17,20 lyase inhibiting compound; and
(ii) an AKT inhibiting compound.

One embodiment of this invention provides a combination comprising:
(i) a 17 α-hydroxylase/C17,20 lyase inhibiting compound; and
(ii) a compound of Structure (II): 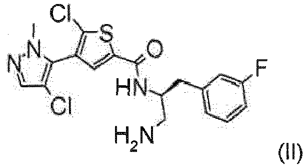 or a pharmaceutically acceptable salt thereof.

One embodiment of this invention provides a combination comprising:
(i) a compound of Structure (I): 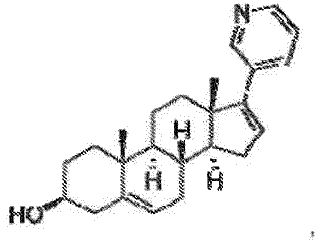 or a pharmaceutically acceptable salt or ester thereof; and
(ii) a compound of Structure (II): 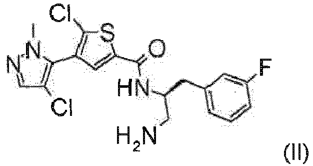 or a pharmaceutically acceptable salt thereof.

One embodiment of this invention provides a method of treating cancer in a human in need thereof which comprises the in vivo administration of a therapeutically effective amount of a 17 α-hydroxylase/C17,20 lyase inhibiting compound; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, and optional additional antineoplastic agents, to such human.

One embodiment of this invention provides a method of treating cancer in a human in need thereof which comprises the in vivo administration of a therapeutically effective amount of a combination of a 17 α-hydroxylase/C17,20 lyase inhibiting compound; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,
wherein the combination is administered within a specified period, and
wherein the combination is administered for a duration of time.

One embodiment of this invention provides a method of treating cancer in a human in need thereof which comprises the in vivo administration of a therapeutically effective amount of a combination of a 17 α-hydroxylase/C17,20 lyase inhibiting compound; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,
wherein the compounds of the combination are administered sequentially.

One embodiment of this invention provides a method of treating cancer in a human in need thereof which comprises the in vivo administration of a therapeutically effective amount of a combination of (3β)-17-(pyridin-3-yl)androsta-5,16-dien-3-ol or a pharmaceutically acceptable salt or ester, suitably the acetate ester, thereof; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, and optional additional antineoplastic agents, to such human.

One embodiment of this invention provides a method of treating cancer in a human in need thereof which comprises the in vivo administration of a therapeutically effective amount of a combination of (3β)-17-(pyridin-3-yl)androsta-5,16-dien-3-ol or a pharmaceutically acceptable salt or ester, suitably the acetate ester, thereof; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,
wherein the combination is administered within a specified period, and
wherein the combination is administered for a duration of time.

One embodiment of this invention provides a method of treating cancer in a human in need thereof which comprises the in vivo administration of a therapeutically effective amount of a combination of (3β)-17-(pyridin-3-yl)androsta-5,16-dien-3-ol or a pharmaceutically acceptable salt or ester, suitably the acetate ester, thereof; and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,
wherein the compounds of the combination are administered sequentially.

### DETAILED OF THE INVENTION

The present invention relates to combinations that exhibit antiproliferative activity. Suitably, the method relates to methods of treating cancer by the co-administration of abiraterone acetate (Zytiga®), (Compound A,
which compound is represented by Structure I: 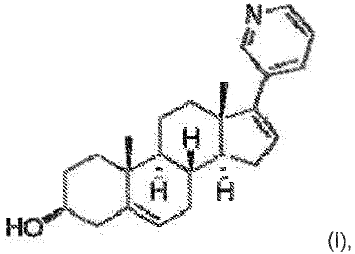 or a pharmaceutically acceptable salt or ester thereof, suitably the acetate ester which is represented by the Structure (III): 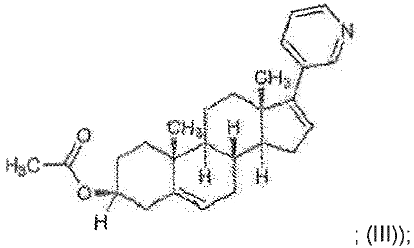 and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, (Compound B is represented by Structure II: 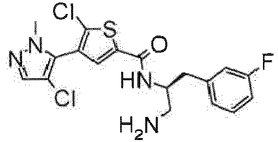 or a pharmaceutically acceptable salt thereof; (II)).

The compound of Structure (I) is sold commercially for the treatment of cancer. The compound of Structure (I) is known by the generic name abiraterone acetate and the trade name Zytiga®.

As used herein the CYP17A1 inhibitor, (3β)-17-(pyridin-3-yl)androsta-5,16-dien-3-ol or a pharmaceutically acceptable salt or ester thereof, is represented by a compound of Structure (I): 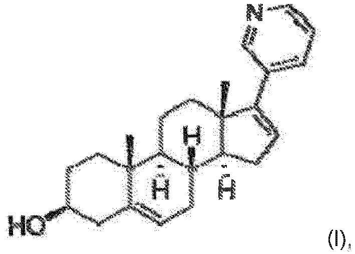 or a pharmaceutically acceptable salt or ester thereof. For convenience, the group of possible compound and salts or esters is collectively referred to as Compound A, meaning that reference to Compound A will refer to any of the compound or pharmaceutically acceptable salt or ester thereof in the alternative.

As used herein, the AKT inhibitor, *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, is represented by a compound of formula (II): 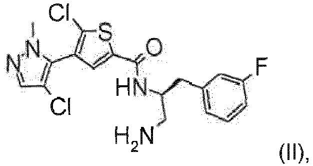 or pharmaceutically acceptable salt thereof. For convenience, the group of possible compound and salts thereof is collectively referred to as Compound B, meaning that reference to Compound B will refer to any of the compound or pharmaceutically acceptable salt thereof in the alternative.

As used herein the term "combination of the invention" refers to a combination comprising a CYP17A1 inhibitor, suitably Compound A and an AKT inhibitor, suitably Compound B.

Compound A is disclosed and claimed, along with pharmaceutically acceptable salts thereof, and also as esters thereof, as being useful as an inhibitor of 17 α-hydroxylase/C17,20 lyase activity, particularly in treatment of cancer, in U.S. Patent No. 5,604,213. Compound A is the compound of Example 2. Compound A can be made by those of skill in the art, suitably as described in U.S. Patent No. 5,604,213.

Compound B is disclosed and claimed, along with pharmaceutically acceptable salts thereof, as being useful as an inhibitor of AKT activity, particularly in treatment of cancer, in International Application No. PCT/US2008/053269, having an International filing date of February 7, 2008; International Publication Number WO 2008/098104 and an International Publication date of August 14, 2008, the entire disclosure of which is hereby incorporated by reference, Compound B is the compound of example 96. Compound B can be prepared as described in International Application No. PCT/US2008/053269.

Suitably, Compound B is in the form of a hydrochloride salt. The salt form can be prepared by one of skill in the art from the description in United States Patent Application Publication: US 2010/0197754 A1, filed 28-Jan-2010, having a publication date of August 5, 2010.

The administration of a therapeutically effective amount of the combinations of the invention are advantageous over the individual component compounds in that the combinations provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides a reduced side effect profile, iv) a reduction in the toxic effect profile, v) an increase in the therapeutic window, or vi) an increase in the bioavailability of one or both of the component compounds.

As used herein the term "neoplasm" refers to an abnormal growth of cells or tissue and is understood to include benign, i.e., non-cancerous growths, and malignant, i.e., cancerous growths. The term "neoplastic" means of or related to a neoplasm.

As used herein the term "agent" is understood to mean a substance that produces a desired effect in a tissue, system, animal, mammal, human, or other subject. Accordingly, the term "anti-neoplastic agent" is understood to mean a substance producing an anti-neoplastic effect in a tissue, system, animal, mammal, human, or other subject. It is also to be understood that an "agent" may be a single compound or a combination or composition of two or more compounds.

By the term "treating" and derivatives thereof as used herein, is meant therapeutic therapy. In reference to a particular condition, treating means: (1) to ameliorate the condition or one or more of the biological manifestations of the condition, (2) to interfere with (a) one or more points in the biological cascade that leads to or is responsible for the condition or (b) one or more of the biological manifestations of the condition (3) to alleviate one or more of the symptoms, effects or side effects associated with the condition or one or more of the symptoms, effects or side effects associated with the condition or treatment thereof, or (4) to slow the progression of the condition or one or more of the biological manifestations of the condition. Prophylactic therapy is also contemplated herein. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. The skilled artisan will appreciate that "prevention" is not an absolute term. Prophylactic therapy is appropriate, for example, when a subject is considered at high risk for developing cancer, such as when a subject has a strong family history of cancer or when a subject has been exposed to high levels of radiation or to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

The compounds of the invention may contain one or more chiral atoms, or may otherwise be capable of existing as enantiomers. Accordingly, the compounds of this invention include mixtures of enantiomers as well as purified enantiomers or enantiomerically enriched mixtures. Also, it is understood that all tautomers and mixtures of tautomers are included within the scope of CYP17A1 inhibiting compounds, suitably Compound A and AKT inhibiting compounds, suitably Compound B.

Also, it is understood that compounds of the presently invented combinations, suitably Compound A and Compound B, may be presented, separately or both, as solvates. As used herein, the term "solvate" refers to a complex of variable stoichiometry formed by a solute (in this invention, compounds of formula (I) or (II) or a salt thereof and a solvent). Such solvents for the purpose of the invention may not interfere with the biological activity of the solute. Examples of suitable solvents include, but are not limited to, water, methanol, ethanol and acetic acid. In one embodiment, the solvent used is a pharmaceutically acceptable solvent. Examples of suitable pharmaceutically acceptable solvents include, without limitation, water, ethanol and acetic acid. In another embodiment, the solvent used is water.

The pharmaceutically acceptable salts of the compounds of the invention are readily prepared by those of skill in the art.

Also, contemplated herein is a method of treating cancer using a combination of the invention where the components, suitably Compound A and/or Compound B or optional additional antineoplastic agents are administered as pro-drugs. Pharmaceutically acceptable pro-drugs of the compounds of the invention are readily prepared by those of skill in the art.

The components of the compositions of the invention, suitably Compounds A and B may have the ability to crystallize in more than one form, a characteristic, which is known polymorphism, and it is understood that such polymorphic forms ("polymorphs") are within the scope of Compounds A and B. Polymorphism generally can occur as a response to changes in temperature or pressure or both and can also result from variations in the crystallization process. Polymorphs can be distinguished by various physical characteristics known in the art such as x-ray diffraction patterns, solubility, and melting point.

Also, contemplated herein is a method of treating cancer using a combination of the invention where components, suitably Compound A and/or Compound B or optional additional antineoplastic agents are administered as pro-drugs. Pharmaceutically acceptable pro-drugs of the compounds of the invention are readily prepared by those of skill in the art.

When referring to a dosing protocol, the term "day", "per day" and the like, refer to a time within one calendar day which begins at midnight and ends at the following midnight.

While it is possible that, for use in therapy, the components of the invention, suitably Compounds A and B, may be administered as the raw chemical, it is preferable to present the active ingredient as a pharmaceutical composition. Accordingly, the invention further provides pharmaceutical compositions, which include a Compound A and/or a Compound B, and one or more pharmaceutically acceptable carriers, diluents, or excipients. The Compounds A and B are as described above. The carrier(s), diluent(s) or excipient(s) must be acceptable in the sense of being compatible with the other ingredients of the formulation, capable of pharmaceutical formulation, and not deleterious to the recipient thereof. In accordance with another aspect of the invention there is also provided a process for the preparation of a pharmaceutical composition including admixing Compound A and/or Compound B, with one or more pharmaceutically acceptable carriers, diluents or excipients. Such elements of the pharmaceutical compositions utilized may be presented in separate pharmaceutical combinations or formulated together in one pharmaceutical composition. Accordingly, the invention further provides a combination of pharmaceutical compositions one of which includes Compound A and one or more pharmaceutically acceptable carriers, diluents, or excipients and a pharmaceutical composition containing Compound B and one or more pharmaceutically acceptable carriers, diluents, or excipients.

The components of the invention, suitably Compound A and Compound B, are as described herein and may be utilized in any of the compositions described above or below.

Pharmaceutical compositions may be presented in unit dose forms containing a predetermined amount of active ingredient per unit dose. As is known to those skilled in the art, the amount of active ingredient per dose will depend on the condition being treated, the route of administration and the age, weight and condition of the patient. Preferred unit dosage compositions are those containing a daily dose or sub-dose, or an appropriate fraction thereof, of an active ingredient. Furthermore, such pharmaceutical compositions may be prepared by any of the methods well known in the pharmacy art. The components of the invention, suitably Compounds A and B, may be administered by any appropriate route. Suitable routes include oral, rectal, nasal, topical (including buccal and sublingual), vaginal, and parenteral (including subcutaneous, intramuscular, intraveneous, intradermal, intrathecal, and epidural). It will be appreciated that the preferred route may vary with, for example, the condition of the recipient of the combination and the cancer to be treated. It will also be appreciated that each of the agents administered may be administered by the same or different routes and that the components, suitably Compounds A and B, may be compounded together in a single pharmaceutical composition.

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also to granulating, the powder mixture can be run through the tablet machine and the result is imperfectly formed slugs broken into granules. The granules can be lubricated be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, compositions for oral administration can be microencapsulated. The composition can also be prepared to prolong or sustain the release as for example by coating or embedding particulate material in polymers, wax or the like.

The agents for use according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents for use according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils.

For treatments of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical compositions adapted for topical administrations to the eye include eye drops wherein the active ingredient is dissolved or suspended in a suitable carrier, especially an aqueous solvent.

Pharmaceutical compositions adapted for topical administration in the mouth include lozenges, pastilles and mouth washes.

Pharmaceutical compositions adapted for rectal administration may be presented as suppositories or as enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists that may be generated by means of various types of metered dose pressurised aerosols, nebulizers or insufflators.

Pharmaceutical compositions adapted for vaginal administration may be presented as pessaries, tampons, creams, gels, pastes, foams or spray compositions.

Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the compositions may include other agents conventional in the art having regard to the type of formulation in question, for example those suitable for oral administration may include flavoring agents.

Unless otherwise defined, in all dosing protocols described herein, the regimen of compounds administered does not have to commence with the start of treatment and terminate with the end of treatment, it is only required that the number of consecutive days in which both compounds are administered and the optional number of consecutive days in which only one of the component compounds is administered, or the indicated dosing protocol - including the amount of compound administered, occur at some point during the course of treatment.

By the term "combination" and derivatives thereof, as used herein is meant either, simultaneous administration or any manner of separate sequential administration of a therapeutically effective amount of Compound A and Compound B. Preferably, if the administration is not simultaneous, the compounds are administered in a close time proximity to each other. Furthermore, it does not matter if the compounds are administered in the same dosage form, e.g. one compound may be administered topically and the other compound may be administered orally. Suitably, both compounds are administered orally.

Thus in one embodiment, one or more doses of Compound A are administered simultaneously or separately with one or more doses of Compound B.

In one embodiment, multiple doses of Compound A are administered simultaneously or separately with multiple doses of Compound B.

In one embodiment, multiple doses of Compound A are administered simultaneously or separately with one dose of Compound B.

In one embodiment, one dose of Compound A is administered simultaneously or separately with multiple doses of Compound B.

In one embodiment one dose of Compound A is administered simultaneously or separately with one dose of Compound B.

In all the above embodiments Compound A may be administered first or Compound B may be administered first.

The combinations may be presented as a combination kit. By the term "combination kit" "or kit of parts" as used herein is meant the pharmaceutical composition or compositions that are used to administer, suitably Compound A and Compound B, according to the invention. When both compounds are administered simultaneously, the combination kit can contain the components, suitably Compound A and Compound B, in a single pharmaceutical composition, such as a tablet, or in separate pharmaceutical compositions. When the components, suitably Compounds A and B are not administered simultaneously, the combination kit will contain the actives in separate pharmaceutical compositions either in a single package or in separate pharmaceutical compositions in separate packages.

In one aspect there is provided a kit of parts comprising:
Compound A in association with pharmaceutically acceptable excipients, diluents and/or carriers; and
Compound B in association with pharmaceutically acceptable excipients, diluents and/or carriers.

In one embodiment of the invention the kit of parts comprises:
Compound A in association with a pharmaceutically acceptable excipients, diluents and/or carriers; and
Compound B in association with a pharmaceutically acceptable excipients, diluents and/or carriers,
wherein the components are provided in a form which is suitable for sequential, separate and/or simultaneous administration.

In one embodiment the kit of parts comprises:
a first container comprising Compound A in association with pharmaceutically acceptable excipients, diluents and/or carriers; and
a second container comprising Compound B in association with pharmaceutically acceptable excipients, diluents and/or carriers, and a container means for containing said first and second containers.

The combination kit can also be provided by instruction, such as dosage and administration instructions. Such dosage and administration instructions can be of the kind that are provided to a doctor, for example by a drug product label, or they can be of the kind that are provided by a doctor, such as instructions to a patient.

In one embodiment of the present invention Compound B is replaced by: 8-[4-(1-aminocyclobutyl)phenyl]-9-phenyl[1,2,4]triazolo[3,4-f]-1,6-naphthyridin-3(2H)-one; which has the following structure (depicted as the chloride salt):

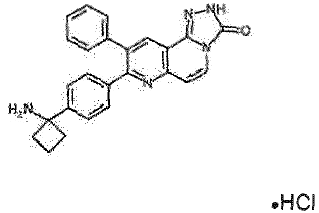

In one embodiment of the present invention Compound B is replaced by:
8-[4-(1-aminocyclobutyl)phenyl]-9-phenyl[1,2,4]triazolo[3,4-f]-1,6-naphthyridin-3(2H)-one or a pharmaceutically acceptable salt thereof.

The compound 8-[4-(1-aminocyclobutyl)phenyl]-9-phenyl[1,2,4]triazolo[3,4-f]-1,6-naphthyridin-3(2H)-one is disclosed and claimed, along with pharmaceutically acceptable salts thereof, as being useful as an inhibitor of AKT activity, particularly in treatment of cancer, in United States Patent 7,576,209 which issued on August 18, 2009. 8-[4-(1-aminocyclobutyl)phenyl]-9-phenyl[1,2,4]triazolo[3,4-f]-1,6-naphthyridin-3(2H)-one can be prepared as described in United States Patent 7,576,209.

In one embodiment of the present invention Compound B is replaced by *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide or a pharmaceutically acceptable salt thereof; which has the following structure (hereinafter referred to as Structure (X)):

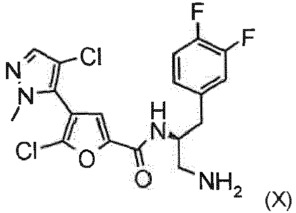

The compound *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide is disclosed and claimed, along with pharmaceutically acceptable salts thereof, as being useful as an inhibitor of AKT activity, particularly in treatment of cancer, in International Application No. PCT/US2008/053269, having an International filing date of February 7, 2008; International Publication Number WO 2008/098104 and an International Publication date of August 14, 2008, the entire disclosure of which is hereby incorporated by reference, *N-*{(1*S*)-2-amino-1-[(3,4-difluorophenyl)methyl]ethyl}-5-chloro-4-(4-chloro-1-methyl-1*H-*pyrazol-5-yl)-2-furancarboxamide is the compound of example 224. *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide can be prepared as described in International Application No. PCT/US2008/053269.

By the term "17 α-hydroxylase/C17,20 lyase inhibitor", "(CYP17A1) inhibitor" and derivatives thereof, as used herein, unless otherwise defined, is meant the class of compounds that suppress testosterone synthesis in castrate and non-castrate males with prostate cancer by inhibiting a key regulatory enzyme of testicular and adrenal androgen synthesis; specifically 17α-hydroxylase/C_{17,20}-lyase (P450c17). Several CYP17A1 inhibitors are marketed or are being studied in the treatment of cancer. In one embodiment of the present invention Compound A is replaced by an alternate CYP17A1 inhibitor. Suitable alternate CYP17A1 inhibitors for use herein include the following.
1. Orteronel, including pharmaceutically acceptable salts thereof. The Journal of Steroid Biochemistry and Molecular Biology 129 (3-5): 115-28 (2012). Orteronel has the following chemical structure and name. 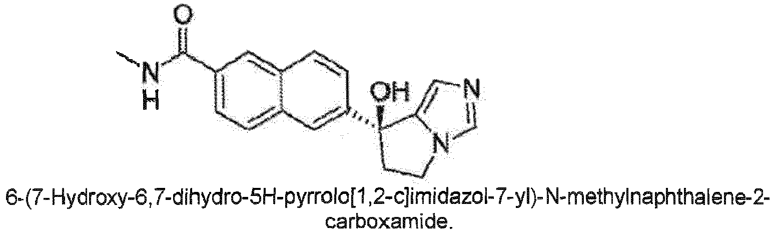
2. Galeterone, including pharmaceutically acceptable salts thereof. "New treatments for castration-resistant prostate cancer: highlights from the 44th annual meeting of the American society of clinical oncology", My 30-June 3, 2008, Chicago, IL. Rev Urol 10 (4): 294-6.
   Galeterone has the following chemical structure and name. 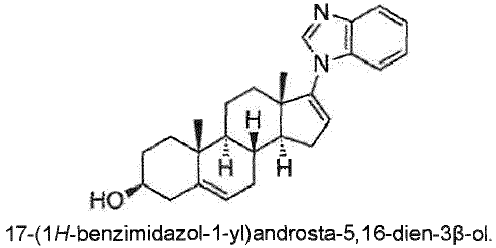
3. CFG920.
4. VT-464.

The term "loading dose" as used herein will be understood to mean a single dose or short duration regimen of a combination of the invention, suitably Compound A and Compound B, having a dosage higher than the maintenance dose administered to the subject to rapidly increase the blood concentration level of the drug. Suitably, a short duration regimen for use herein will be from: 1 to 14 days; suitably from 1 to 7 days; suitably from 1 to 3 days; suitably for three days; suitably for two days; suitably for one day. In some embodiments, the "loading dose" can increase the blood concentration of the drug to a therapeutically effective level. In some embodiments, the "loading dose" can increase the blood concentration of the drug to a therapeutically effective level in conjunction with a maintenance dose of the drug. The "loading dose" can be administered once per day, or more than once per day (e.g., up to 4 times per day). Suitably the "loading dose" will be administered once a day. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading dose will be administered for from 1 to 7 days: suitably from 1 to 5 days; suitably from 1 to 3 days; suitably for 1 day; suitably for 2 days; suitably for 3 days, followed by a maintenance dosing protocol.

The term "maintenance dose" as used herein will be understood to mean a dose that is serially administered (for example; at least twice), and which is intended to either slowly raise blood concentration levels of the compound to a therapeutically effective level, or to maintain such a therapeutically effective level. The maintenance dose can be administered once per day and the maintenance dose is lower than the loading dose.

Suitably the combinations of this invention are administered within a "specified period".

By the term "specified period" and derivatives thereof, as used herein is meant the interval of time between the administration of one component of the invention, suitably Compound A or Compound B, and the other of the components, suitably the other of Compound A and Compound B. Unless otherwise defined, the specified period can include simultaneous administration. When two compounds of the invention are administered once a day the specified period refers to administration of both components, suitably Compound A and Compound B, during a single day. When one or both compounds of the invention are administered more than once a day, the specified period is calculated based on the first administration of each compound on a specific day. All administrations of a compound of the invention that are subsequent to the first during a specific day are not considered when calculating the specific period.

Suitably, if the compounds are administered within a "specified period" and not administered simultaneously, they are both administered within about 24 hours of each other - in this case, the specified period will be about 24 hours; suitably they will both be administered within about 12 hours of each other - in this case, the specified period will be about 12 hours; suitably they will both be administered within about 11 hours of each other - in this case, the specified period will be about 11 hours; suitably they will both be administered within about 10 hours of each other - in this case, the specified period will be about 10 hours; suitably they will both be administered within about 9 hours of each other - in this case, the specified period will be about 9 hours; suitably they will both be administered within about 8 hours of each other - in this case, the specified period will be about 8 hours; suitably they will both be administered within about 7 hours of each other - in this case, the specified period will be about 7 hours; suitably they will both be administered within about 6 hours of each other - in this case, the specified period will be about 6 hours; suitably they will both be administered within about 5 hours of each other - in this case, the specified period will be about 5 hours; suitably they will both be administered within about 4 hours of each other - in this case, the specified period will be about 4 hours; suitably they will both be administered within about 3 hours of each other - in this case, the specified period will be about 3 hours; suitably they will be administered within about 2 hours of each other - in this case, the specified period will be about 2 hours; suitably they will both be administered within about 1 hour of each other - in this case, the specified period will be about 1 hour. As used herein, the administration of Compound A and Compound B in less than about 45 minutes apart is considered simultaneous administration.

Suitably, when the combination of the invention is administered for a "specified period", the compounds will be co-administered for a "duration of time".

By the term "duration of time" and derivatives thereof, as used herein is meant that both compounds of the invention are administered for an indicated number of consecutive days.

Regarding "specified period" administration:
Suitably, both compounds will be administered within a specified period for at least one day - in this case, the duration of time will be at least one day; suitably, during the course to treatment, both compounds will be administered within a specified period for at least 3 consecutive days - in this case, the duration of time will be at least 3 days; suitably, during the course to treatment, both compounds will be administered within a specified period for at least 5 consecutive days - in this case, the duration of time will be at least 5 days; suitably, during the course to treatment, both compounds will be administered within a specified period for at least 7 consecutive days - in this case, the duration of time will be at least 7 days; suitably, during the course to treatment, both compounds will be administered within a specified period for at least 14 consecutive days - in this case, the duration of time will be at least 14 days; suitably, during the course to treatment, both compounds will be administered within a specified period for at least 30 consecutive days - in this case, the duration of time will be at least 30 days. When, during the course of treatment, both compounds are administered within a specified period for over 30 days, the treatment is considered chronic treatment and will continue until an altering event, such as a reassessment in cancer status or a change in the condition of the patient, warrants a modification to the protocol.

Further regarding "specified period" administration:
Suitably during the course of treatment, the components, suitably Compound A and Compound B, will be administered within a specified period for from 1 to 4 days over a 7 day period, and during the other days of the 7 day period the CYP17A1 inhibiting compound, suitably Compound A, will be administered alone. Suitably, this 7 day protocol is repeated for 2 cycles or for 14 days; suitably for 4 cycles or 28 days; suitably for continuous administration.

Suitably, during the course of treatment a combination of the invention, suitably Compound A and Compound B, will be administered within a specified period for from 1 to 4 days over a 7 day period, and during the other days of the 7 day period the AKT inhibiting compound, suitably Compound B, will be administered alone. Suitably, this 7 day protocol is repeated for 2 cycles or for 14 days; suitably for 4 cycles or 28 days; suitably for continuous administration. The AKT inhibiting compound, suitably Compound B, is administered for consecutive days during the 7 day period. Suitably the AKT inhibitingcompound, suitably Compound B, is administered in a pattern of every other day during each 7 day period.

Suitably, during the course of treatment a combination of the invention, suitably Compound A and Compound B, will be administered within a specified period for 3 days over a 7 day period, and during the other days of the 7 day period the AKT inhibiting compound, suitably Compound B, will be administered alone. Suitably, this 7 day protocol is repeated for 2 cycles or for 14 days; suitably for 4 cycles or 28 days; suitably for continuous administration. Suitably the CYP17A1 inhibiting compound, suitably Compound A will be administered 3 consecutive days during the 7 day period.

Suitably, during the course of treatment a combination of the invention, suitably Compound A and Compound B, will be administered within a specified period for 2 days over a 7 day period, and during the other days of the 7 day period the AKT inhibiting compound, suitably Compound B, will be administered alone. Suitably, this 7 day protocol is repeated for 2 cycles or for 14 days; suitably for 4 cycles or 28 days; suitably for continuous administration. Suitably, the CYP17A1 inhibiting compound, suitably Compound A, will be administered 2 consecutive days during the 7 day period.

Suitably, during the course of treatment a combination of the invention, suitably Compound A and Compound B, will be administered within a specified period for 1 day during a 7 day period, and during the other days of the 7 day period the AKT inhibiting compound, suitably Compound B, will be administered alone. Suitably, this 7 day protocol is repeated for 2 cycles or for 14 days; suitably for 4 cycles or 28 days; suitably for continuous administration.

Suitably, if the compounds are not administered during a "specified period", they are administered sequentially. By the term "sequential administration", and derivates thereof, as used herein is meant that one component of the invention, suitably Compound A or Compound B, is administered for two or more consecutive days and the other component of the invention, suitably the other of Compound A and Compound B, is subsequently administered for two or more consecutive days. Also, contemplated herein is a drug holiday utilized between the sequential administration of a combination of the invention. As used herein, a drug holiday is a period of days after the sequential administration of a combination of the invention, suitably of Compound A and Compound B and before the administration of another combination of the invention, suitably ofCompound A and Compound B, where no compound is administered. Suitably the drug holiday will be a period of days selected from: 1 day, 2 days, 3 days, 4 days, 5 days, 6 days, 7 days, 8 days, 9 days, 10 days, 11 days, 12 days, 13 days and 14 days.

Regarding sequential administration:
Suitably one component of the invention, suitably one of Compound A and Compound B, is administered for from 1 to 30 consecutive days, followed by an optional drug holiday, followed by administration of the other component of the invention, suitably the other of Compound A and Compound B, for from 1 to 30 consecutive days. Suitably, one of Compound A and Compound B is administered for from 2 to 21 consecutive days, followed by an optional drug holiday, followed by administration of the other of Compound A and Compound B for from 2 to 21 consecutive days. Suitably, one of Compound A and Compound B is administered for from 2 to 14 consecutive days, followed by a drug holiday of from 1 to 14 days, followed by administration of the other of Compound A and Compound B for from 2 to 14 consecutive days. Suitably, one of Compound A and Compound B is administered for from 3 to 7 consecutive days, followed by a drug holiday of from 3 to 10 days, followed by administration of the other of Compound A and Compound B for from 3 to 7 consecutive days.

Suitably, Compound B will be administered first in the sequence, followed by an optional drug holiday, followed by administration of Compound A. Suitably, Compound B is administered for from 1 to 21 consecutive days, followed by an optional drug holiday, followed by administration of Compound A for from 1 to 21 consecutive days. Suitably, Compound B is administered for from 3 to 21 consecutive days, followed by a drug holiday of from 1 to 14 days, followed by administration of Compound A for from 3 to 21 consecutive days. Suitably, Compound B is administered for from 3 to 21 consecutive days, followed by a drug holiday of from 3 to 14 days, followed by administration of Compound A for from 3 to 21 consecutive days. Suitably, Compound B is administered for 21 consecutive days, followed by an optional drug holiday, followed by administration of Compound A for 14 consecutive days. Suitably, Compound B is administered for 14 consecutive days, followed by a drug holiday of from 1 to 14 days, followed by administration of Compound A for 14 consecutive days. Suitably, Compound B is administered for 7 consecutive days, followed by a drug holiday of from 3 to 10 days, followed by administration of Compound A for 7 consecutive days. Suitably, Compound B is administered for 3 consecutive days, followed by a drug holiday of from 3 to 14 days, followed by administration of Compound A for 7 consecutive days. Suitably, Compound B is administered for 3 consecutive days, followed by a drug holiday of from 3 to 10 days, followed by administration of Compound A for 3 consecutive days.

Suitably, Compound A will be administered first in the sequence, followed by an optional drug holiday, followed by administration of Compound B. Suitably, Compound A is administered for from 1 to 21 consecutive days, followed by an optional drug holiday, followed by administration of Compound B for from 1 to 21 consecutive days. Suitably, Compound A is administered for from 3 to 21 consecutive days, followed by a drug holiday of from 1 to 14 days, followed by administration of Compound B for from 3 to 21 consecutive days. Suitably, Compound A is administered for from 3 to 21 consecutive days, followed by a drug holiday of from 3 to 14 days, followed by administration of Compound B for from 3 to 21 consecutive days. Suitably, Compound A is administered for 21 consecutive days, followed by an optional drug holiday, followed by administration of Compound B for 14 consecutive days. Suitably, Compound A is administered for 14 consecutive days, followed by a drug holiday of from 1 to 14 days, followed by administration of Compound B for 14 consecutive days. Suitably, Compound A is administered for 7 consecutive days, followed by a drug holiday of from 3 to 10 days, followed by administration of Compound B for 7 consecutive days. Suitably, Compound A is administered for 3 consecutive days, followed by a drug holiday of from 3 to 14 days, followed by administration of Compound B for 7 consecutive days. Suitably, Compound A is administered for 3 consecutive days, followed by a drug holiday of from 3 to 10 days, followed by administration of Compound B for 3 consecutive days.

It is understood that a "specified period" administration and a "sequential" administration can be followed by repeat dosing or can be followed by an alternate dosing protocol, and a drug holiday may precede the repeat dosing or alternate dosing protocol.

Suitably, the amount of Compound A (based on weight of acetate ester amount) administered as part of the combination according to the present invention will be an amount selected from about 1mg to about 1000mg; suitably, the amount will be selected from about 100mg to about 900mg; suitably, the amount will be selected from about 200mg to about 800mg; suitably, the amount will be selected from about 300mg to about 700mg; suitably, the amount will be selected from about 400mg to about 600mg; suitably, the amount will be about 500mg; suitably, the amount will be about 250mg. Suitably the amount will be 1000mg administered in 4 250mg doses. Accordingly, the amount of Compound A administered as part of the combination according to the present invention will be an amount selected from about 100mg to about 1000mg. For example, the amount of Compound A administered as part of the combination according to the present invention can be 1000mg, 900mg, 800mg, 700mg, 600mg, 500mg,400mg, 300mg, 250mg, 200mg, 100mg.

Suitably, the selected amount of Compound A is administered daily. Suitably, the selected amount of Compound A is administered twice a day. Suitably, the selected amount of Compound A is administered from 1 to 5 times a day. Suitably, Compound A is administered at an amount of 250mg administered 4 times a day. Suitably, the Compound A will be administered in a load dose.

Suitably, the amount of Compound B (based on weight of free base amount) administered as part of the combination according to the present invention will be an amount selected from about 1mg to about 500mg; suitably, the amount will be selected from about 5mg to about 400mg; suitably, the amount will be selected from about 30mg to about 375mg; suitably, the amount will be selected from about 35mg to about 350mg; suitably, the amount will be selected from about 40mg to about 300mg; suitably, the amount will be selected from about 45mg to about 275mg; suitably, the amount will be selected from about 50mg to about 250mg; suitably, the amount will be selected from about 55mg to about 225mg; suitably, the amount will be selected from about 60mg to about 200mg; suitably, the amount will be selected from about 65mg to about 175mg; suitably, the amount will be selected from about 70mg to about 150mg; suitably, the amount will be selected from about 50mg to about 300mg; suitably, the amount will be selected from about 75mg to about 150mg; suitably, the amount will be about 100mg. Accordingly, the amount of Compound B administered as part of the combination according to the present invention will be an amount selected from about 5mg to about 500mg. For example, the amount of Compound B administered as part of the combination according to the present invention can be 5mg, 10mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 105mg, 110mg, 115mg, 120mg, 125mg, 130mg, 135mg, 140mg, 145mg, 150mg, 175mg, 200mg, 225mg, 250mg, 275mg, 300mg, 325mg, 350mg, 375mg, 400mg, 425mg, 450mg, 475mg or 500mg. Suitably, the selected amount of Compound B is administered twice a day. Suitably, the selected amount of Compound B² is administered once a day. Suitably, the administration of Compound B will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

As used herein, the amounts specified for Compound A and Compound B, unless otherwise defined, are indicated as the amount of free or unsalted compound.

### METHOD OF TREATMENT

The combinations of the invention are believed to have utility in disorders wherein the inhibition of AKT and/or 17 α-hydroxylase/C17,20 lyase (CYP17A1) is beneficial.

The present invention thus also provides a combination of the invention, for use in therapy, particularly in the treatment of disorders wherein the inhibition of AKT and/or 17 α-hydroxylase/C17,20 lyase (CYP17A1) activity is beneficial, particularly cancer, particularly prostate cancer.

A further aspect of the invention provides a method of treatment of a disorder wherein to inhibition of AKT and/or 17 α-hydroxylase/C17,20 lyase (CYP17A1) is beneficial, comprising administering a combination of the invention.

A further aspect of the present invention provides the use of a combination of the invention in the manufacture of a medicament for the treatment of a disorder wherein the inhibition of AKT and/or 17 α-hydroxylase/C17,20 lyase (CYP17A1) is beneficial.

Typically, the disorder is a cancer such that inhibition of AKT and/or 17 α-hydroxylase/C17,20 lyase (CYP17A1) has a beneficial effect. Examples of cancers that are suitable for treatment with combination of the invention include, but are limited to, both primary and metastatic forms of head and neck, breast, lung, colon, ovary, and prostate cancers. Suitably the cancer is selected from: brain (gliomas), glioblastomas, astrocytomas, glioblastoma multiforme, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, melanoma, ovarian, pancreatic, prostate, sarcoma, osteosarcoma, giant cell tumor of bone, thyroid cancer, lymphoblastic T cell leukemia, Chronic myelogenous leukemia, Chronic lymphocytic leukemia, Hairy-cell leukemia, acute lymphoblastic leukemia, acute myelogenous leukemia, AML, Chronic neutrophilic leukemia, Acute lymphoblastic T cell leukemia, plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Additionally, examples of a cancer to be treated include Barret's adenocarcinoma; billiary tract carcinomas; breast cancer; cervical cancer; cholangiocarcinoma; central nervous system tumors including primary CNS tumors such as glioblastomas, astrocytomas (e.g., glioblastoma multiforme) and ependymomas, and secondary CNS tumors (i.e., metastases to the central nervous system of tumors originating outside of the central nervous system); colorectal cancer including large intestinal colon carcinoma; gastric cancer; carcinoma of the head and neck including squamous cell carcinoma of the head and neck; hematologic cancers including leukemias and lymphomas such as acute lymphoblastic leukemia, acute myelogenous leukemia (AML), myelodysplastic syndromes, chronic myelogenous leukemia, Hodgkin's lymphoma, non-Hodgkin's lymphoma, megakaryoblastic leukemia, multiple myeloma and erythroleukemia; hepatocellular carcinoma; lung cancer including small cell lung cancer and non-small cell lung cancer; ovarian cancer; endometrial cancer; pancreatic cancer; pituitary adenoma; prostate cancer; renal cancer; sarcoma; skin cancers including melanomas; and thyroid cancers.

In one embodiment, the cancer described here is PTEN deficient. As used herein, the phrase "PTEN deficient" or "PTEN deficiency" shall describe tumors with deficiencies in the function of the tumor suppressor PTEN (Phosphatase and Tensin Homolog). Such deficiency can include one or more of the following: i.) point mutation in the PTEN gene, ii.) reduction or absence of PTEN proteins when compared to PTEN wild-type, iii.) mutation or absence of other genes that cause suppression of PTEN function,iv.) partial or full gene deletions, and/or v.) epigenetic modification of the PTEN promoter or gene in such a way that it silences expression of the PTEN gene.

Suitably, the present invention relates to a method for treating or lessening the severity of a cancer selected from: brain (gliomas), glioblastomas, Bannayan-Zonana syndrome, Cowden disease, Lhermitte-Duclos disease, breast, colon, head and neck, kidney, lung, liver, melanoma, ovarian, pancreatic, prostate, sarcoma and thyroid.

Suitably, the present invention relates to a method for treating or lessening the severity of a cancer selected from ovarian, breast, pancreatic and prostate.

Suitably, the present invention relates to a method for treating or lessening the severity of prostate cancer.

The combination of the invention may be used alone or in combination with one or more other therapeutic agents. The invention thus provides in a further aspect a further combination comprising a combination of the invention with a further therapeutic agent or agents, compositions and medicaments comprising the combination and use of the further combination, compositions and medicaments in therapy, in particular in the treatment of diseases susceptible to inhibition of AKT and/or 17 α-hydroxylase/C17,20 lyase (CYP17A1).

In the embodiment, the combination of the invention may be employed with other therapeutic methods of cancer treatment. In particular, in anti-neoplastic therapy, combination therapy with other chemotherapeutic, hormonal, antibody agents as well as surgical and/or radiation treatments other than those mentioned above are envisaged. Combination therapies according to the present invention thus include the administration of Compound A and Compound B as well as optional use of other therapeutic agents including other anti-neoplastic agents. Such combination of agents may be administered together or separately and, when administered separately this may occur simultaneously or sequentially in any order, both close and remote in time. In one embodiment, the pharmaceutical combination includes Compound A and Compound B, and optionally at least one additional anti-neoplastic agent.

As indicated, therapeutically effective amounts of Compound A and Compound B are discussed above. The therapeutically effective amount of the further therapeutic agents of the present invention will depend upon a number of factors including, for example, the age and weight of the patient, the precise condition requiring treatment, the severity of the condition, the nature of the formulation, the nature of the disease under treatment, and the route of administration. Ultimately, the therapeutically effective amount will be at the discretion of the attendant physician. The relative timings of administration will be selected in order to achieve the desired combined therapeutic effect.

In one embodiment, the further anti-cancer therapy is surgical and/or radiotherapy.

In one embodiment, the further anti-cancer therapy is at least one additional anti-neoplastic agent.

Any anti-neoplastic agent that has activity versus a susceptible tumor being treated may be utilized in the combination. Typical anti-neoplastic agents useful include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine angiogenesis inhibitors; immunotherapeutic agents; proapoptotic agents; late stage development drug treatments including conjugates which are antibodies against prostate cancer targets that are chemically conjugated to potent microtubule inhibitors such as monomethylauristatin E (MMAE) and the maytansinoids (DM1, DM4), or DNA binding agents such as the pyrrolobenzodiazepine dimmers; and cell cycle signaling inhibitors.

Cabazitaxel, 2aR,4S,4aS,6R,9S,11S,12S,12aR,12bS)-12b-acetoxy-9-(((2R,3S)-3-((tert-butoxycarbonyl)amino)-2-hydroxy-3-phenylpropanoyl)oxy)-11-hydroxy-4,6-dimethoxy-4a,8,13,13-tetramethyl-5-oxo-2a,3,4,4a,5,6,9,10,11,12,12a,12b-dodecahydro-1H-7,11-methanocyclodeca[3,4]benzo[1,2-b]oxet-12-yl benzoate is a treatment option for hormone-refractory prostate cancer. Cabazitaxel is a semi-synthetic derivative of the natural taxoid 10-deacetylbaccatin III with potential antineoplastic activity. Cabazitaxel binds to and stabilizes tubulin, resulting in the inhibition of microtubule depolymerization and cell division, cell cycle arrest in the G2/M phase, and the inhibition of tumor cell proliferation.

Anti-microtubule or anti-mitotic agents: Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti - cancer agents that operate at the G₂/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree *Taxus brevifolia* and is commercially available as an injectable solution TAXOL®. It is a member of the taxane family of terpenes. Paclitaxel has been approved for clinical use in the treatment of refractory ovarian cancer in the United States (Markman et al., Yale Journal of Biology and Medicine, 64:583, 1991; McGuire et al., Ann. Intem, Med., 111:273, 1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide, 1998) related to the duration of dosing above a threshold concentration (50nM) (Kearns, C.M. et. al., Seminars in Oncology, 3(6) p.16-23, 1995).

Docetaxel, (2R,3S)- N-carboxy-3-phenylisoserine,N-*tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel *q.v.,* prepared using a natural precursor, 10-deacetyl-baccatin III, extracted from the needle of the European Yew tree.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro-4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes: Platinum coordination complexes are non-phase specific anti-cancer agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, oxaliplatin, cisplatin and carboplatin.

Cisplatin, cis-diamminedichloroplatinum, is commercially available as PLATINOL® as an injectable solution. Cisplatin is primarily indicated in the treatment of metastatic testicular and ovarian cancer and advanced bladder cancer.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced ovarian carcinoma.

Alkylating agents: Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease.

Busulfan, 1,4-butanediol dimethanesulfonate, is commercially available as MYLERAN® TABLETS. Busulfan is indicated for the palliative treatment of chronic myelogenous leukemia.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease.

Antibiotic anti-neoplastics: Antibiotic anti-neoplastics are non-phase specific agents, which bind or intercalate with DNA. Typically such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also know as Actinomycin D, is commercially available in injectable form as COSMEGEN®. Dactinomycin is indicated for the treatment of Wilm's tumor and rhabdomyosarcoma.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas.

Bleomycin, a mixture of cytotoxic glycopeptide antibiotics isolated from a strain of *Streptomyces verticillus,* is commercially available as BLENOXANE®. Bleomycin is indicated as a palliative treatment, as a single agent or in combination with other agents, of squamous cell carcinoma, lymphomas, and testicular carcinomas.

Topoisomerase II inhibitors: Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as a single agent or in combination with other chemotherapy agents in the treatment of testicular and non-small cell lung cancers.

Teniposide, 4'-demethyl-epipodophyllotoxin 9[4,6-0-(R)-thenylidene-β-D-glucopyranoside], is commercially available as an injectable solution as VUMON® and is commonly known as VM-26. Teniposide is indicated as a single agent or in combination with other chemotherapy agents in the treatment of acute leukemia in children.

Antimetabolite neoplastic agents: Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Other fluoropyrimidine analogs include 5-fluoro deoxyuridine (floxuridine) and 5-fluorodeoxyuridine monophosphate.

Cytarabine, 4-amino-1-β-D-arabinofuranosyl-2 (1H)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine).

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. A useful mercaptopurine analog is azathioprine.

Thioguanine, 2-amino-1,7-dihydro-6H-purine-6-thione, is commercially available as TABLOID®. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Other purine analogs include pentostatin, erythrohydroxynonyladenine, fludarabine phosphate, and cladribine.

Gemcitabine, 2'-deoxy-2',2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer.

Methotrexate, N-[4[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder.

Topoisomerase I inhibitors: Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCI, (4S)-4,11-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR®. Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA : irintecan or SN-38 ternary complex with replication enzymes. Irinotecan is indicated for treatment of metastatic cancer of the colon or rectum.

Topotecan HCI, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer.

Hormones and hormonal analogues: Hormones and hormonal analogues are useful compounds for treating cancers in which there is a relationship between the hormone(s) and growth and/or lack of growth of the cancer. Examples of hormones and hormonal analogues useful in cancer treatment include, but are not limited to, adrenocorticosteroids such as prednisone and prednisolone which are useful in the treatment of malignant lymphoma and acute leukemia in children ; aminoglutethimide and other aromatase inhibitors such as anastrozole, letrazole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and anti-androgens such as flutamide, nilutamide, bicalutamide, cyproterone acetate and 5α-reductases such as finasteride and dutasteride, useful in the treatment of prostatic carcinoma and benign prostatic hypertrophy; anti-estrogens such as tamoxifen, toremifene, raloxifene, droloxifene, iodoxyfene, as well as selective estrogen receptor modulators (SERMS) such those described in U.S. Patent Nos. 5,681,835, 5,877,219, and 6,207,716, useful in the treatment of hormone dependent breast carcinoma and other susceptible cancers; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

Signal transduction pathway inhibitors: Signal transduction pathway inhibitors are those inhibitors, which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3domain blockers, serine/threonine kinases, phosphotidyl inositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are generally termed growth factor receptors. Inappropriate or uncontrolled activation of many of these kinases, i.e. aberrant kinase growth factor receptor activity, for example by over-expression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr), platelet derived growth factor receptor (PDGFr), erbB2, erbB4, ret, vascular endothelial growth factor receptor (VEGFr), tyrosine kinase with immunoglobulin-like and epidermal growth factor homology domains (TIE-2), insulin growth factor -I (IGFI) receptor, macrophage colony stimulating factor (cfms), BTK, ckit, cmet, fibroblast growth factor (FGF) receptors, Trk receptors (TrkA, TrkB, and TrkC), ephrin (eph) receptors, and the RET protooncogene. Several inhibitors of growth receptors are under development and include ligand antagonists, antibodies, tyrosine kinase inhibitors and anti-sense oligonucleotides. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth factor receptors as targets", New Molecular Targets for Cancer Chemotherapy, ed. Workman, Paul and Kerr, David, CRC press 1994, London.

Tyrosine kinases, which are not growth factor receptor kinases are termed non-receptor tyrosine kinases. Non-receptor tyrosine kinases useful in the present invention, which are targets or potential targets of anti-cancer drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997) Annual review of Immunology. 15: 371-404.

SH2/SH3 domain blockers are agents that disrupt SH2 or SH3 domain binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (rafk), Mitogen or Extracellular Regulated Kinase (MEKs), and Extracellular Regulated Kinases (ERKs); and Protein kinase C family member blockers including blockers of PKCs (alpha, beta, gamma, epsilon, mu, lambda, iota, zeta). IkB kinase family (IKKa, IKKb), PKB family kinases, akt kinase family members, and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60. 1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; U.S. Patent No. 6,268,391; and Martinez-lacaci, L., et al, Int. J. Cancer (2000), 88(1), 44-52.

Inhibitors of Phosphotidyl inositol-3 Kinase family members including blockers of PI3-kinase, ATM, DNA-PK, and Ku are also useful in the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997), International Journal of Biochemistry and Cell Biology. 29 (7):935-8; and Zhong, H. et al, Cancer res, (2000) 60(6), 1541-1545.

Also useful in the present invention are Myo-inositol signaling inhibitors such as phospholipase C blockers and Myoinositol analogues. Such signal inhibitors are described in Powis, G., and Kozikowski A., (1994) New Molecular Targets for Cancer Chemotherapy ed., Paul Workman and David Kerr, CRC press 1994, London.

Another group of signal transduction pathway inhibitors are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block ras activation in cells containing wild type mutant ras , thereby acting as antiproliferation agents. Ras oncogene inhibition is discussed in Scharovsky, O.G., Rozados, V.R., Gervasoni, S.I. Matar, P. (2000), Journal of Biomedical Science. 7(4) 292-8; Ashby, M.N. (1998), Current Opinion in Lipidology. 9 (2) 99 - 102; and BioChim. Biophys. Acta, (19899) 1423(3):19-30.

As mentioned above, antibody antagonists to receptor kinase ligand binding may also serve as signal transduction inhibitors. This group of signal transduction pathway inhibitors includes the use of humanized antibodies to the extracellular ligand binding domain of receptor tyrosine kinases. For example Imclone C225 EGFR specific antibody (see Green, M.C. et al, Monoclonal Antibody Therapy for Solid Tumors, Cancer Treat. Rev., (2000), 26(4), 269-286); Herceptin ® erbB2 antibody (see Tyrosine Kinase Signalling in Breast cancer:erbB Family Receptor Tyrosine Kinases, Breast cancer Res., 2000, 2(3), 176-183); and 2CB VEGFR2 specific antibody (see Brekken, R.A. et al, Selective Inhibition of VEGFR2 Activity by a monoclonal Anti-VEGF antibody blocks tumor growth in mice, Cancer Res. (2000) 60, 5117-5124).

Anti-angiogenic agents: Anti-angiogenic agents including non-receptorMEKngiogenesis inhibitors may alo be useful. Anti-angiogenic agents such as those which inhibit the effects of vascular edothelial growth factor, (for example the anti-vascular endothelial cell growth factor antibody bevacizumab [Avastin™], and compounds that work by other mechanisms (for example linomide, inhibitors of integrin αvβ3 function, endostatin and angiostatin);

Immunotherapeutic agents: Agents used in immunotherapeutic regimens may also be useful in combination with the compounds of formula (I). Immunotherapy approaches, including for example ex-vivo and in-vivo approaches to increase the immunogenecity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies

Proapoptotoc agents: Agents used in proapoptotic regimens (e.g., bcl-2 antisense oligonucleotides) may also be used in the combination of the present invention.

Cell cycle signalling inhibitors: Cell cycle signalling inhibitors inhibit molecules involved in the control of the cell cycle. A family of protein kinases called cyclin dependent kinases (CDKs) and their interaction with a family of proteins termed cyclins controls progression through the eukaryotic cell cycle. The coordinate activation and inactivation of different cyclin/CDK complexes is necessary for normal progression through the cell cycle. Several inhibitors of cell cycle signalling are under development. For instance, examples of cyclin dependent kinases, including CDK2, CDK4, and CDK6 and inhibitors for the same are described in, for instance, Rosania et al, Exp. Opin. Ther. Patents (2000) 10(2):215-230. Further, p21WAF1/CIP1 has been described as a potent and universal inhibitor of cyclin-dependent kinases (Cdks) (Ball et al., Progress in Cell Cycle Res., 3: 125 (1997)). Compounds that are known to induce expression of p21WAF1/CIP1 have been implicated in the suppression of cell proliferation and as having tumor suppressing activity (Richon et al., Proc. Nat Acad. Sci. U.S.A. 97(18): 10014-10019 (2000)), and are included as cell cycle signaling inhibitors. Histone deacetylase (HDAC) inhibitors are implicated in the transcriptional activation of p21WAF1/CIP1 (Vigushin et al., Anticancer Drugs, 13(1): 1-13 (Jan 2002)), and are suitable cell cycle signaling inhibitors for use in combination herein.

Examples of such HDAC inhibitors include:
1. Vorinostat, including pharmaceutically acceptable salts thereof. Marks et al., Nature Biotechnology 25, 84 to 90 (2007); Stenger, Community Oncology 4, 384-386 (2007).
   Vorinostat has the following chemical structure and name: 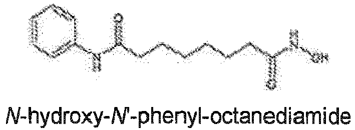
2. Romidepsin, including pharmaceutically acceptable salts thereof. Vinodhkumar et al., Biomedicine & Pharmacotherapy 62 (2008) 85-93. Romidepsin, has the following chemical structure and name: 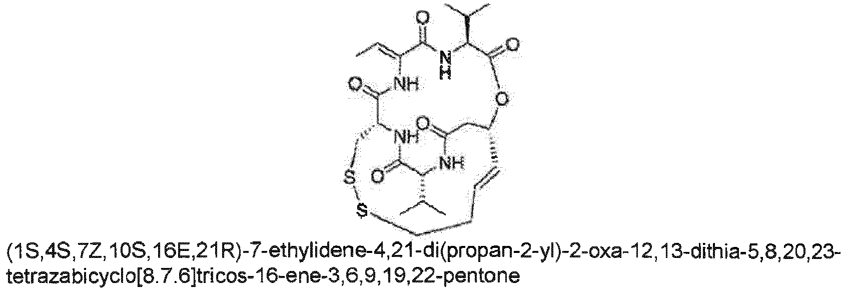
3. Panobinostat, including pharmaceutically acceptable salts thereof. Drugs of the Future 32(4): 315-322 (2007*).*
   Panobinostat, has the following chemical structure and name: 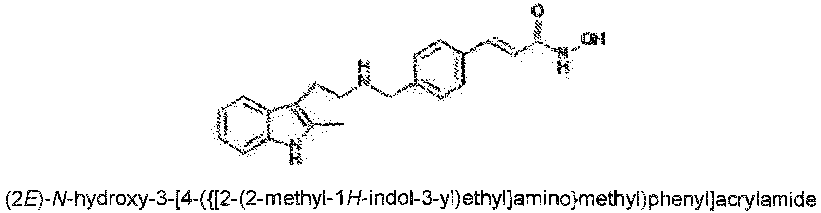
4. Valproic acid, including pharmaceutically acceptable salts thereof. Gottlicher, et al., EMBO J. 20(24): 6969-6978 (2001).
   Valproic acid, has the following chemical structure and name: 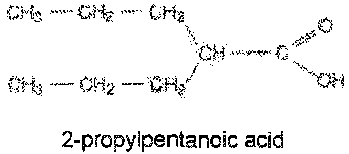
5. Mocetinostat (MGCD0103), including pharmaceutically acceptable salts thereof. Balasubramanian et al., Cancer Letters 280: 211-221 (2009).
   Mocetinostat, has the following chemical structure and name:

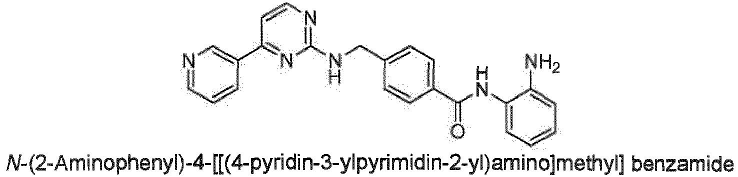

Further examples of such HDAC inhibitors are included in Bertrand European Journal of Medicinal Chemistry 45, (2010) 2095-2116, particularly the compounds of table 3 therein as indicated below.

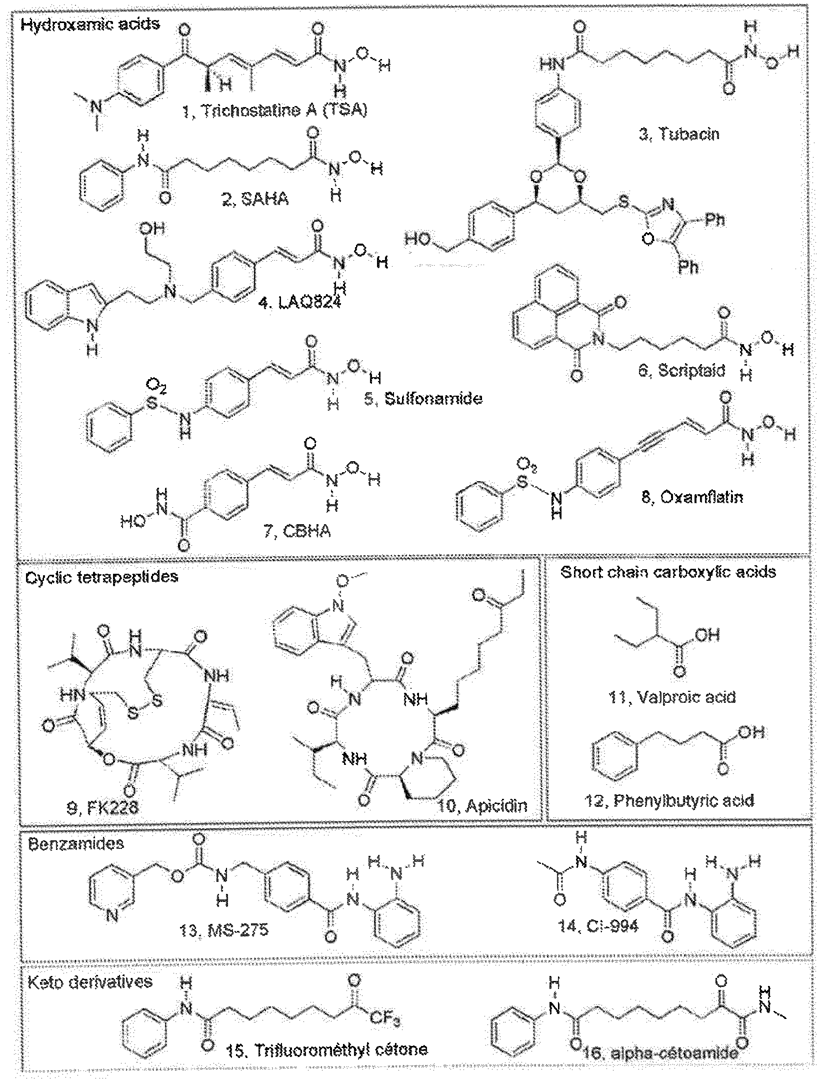

Proteasome inhibitors are drugs that block the action of proteasome, cellular complexes that break down proteins, like the p53 protein. Several proteasome inhibitors are marketed or are being studied in the treatment of cancer. Suitable proteasome inhibitors for use in combination herein include:
1. Bortezomib (Velcade®), including pharmaceutically acceptable salts thereof. Adams J, Kauffman M (2004), Cancer Invest 22 (2): 304-11. Bortezomib has the following chemical structure and name. 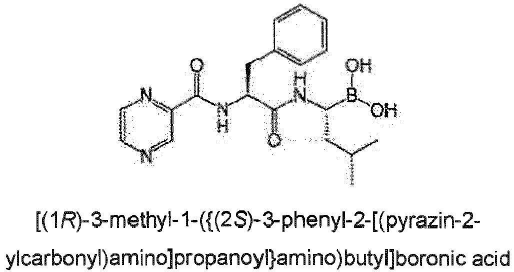
2. Disulfiram, including pharmaceutically acceptable salts thereof. Bouma et al. (1998). J. Antimicrob. Chemother. 42 (6): 817-20. Disulfiram has the following chemical structure and name. 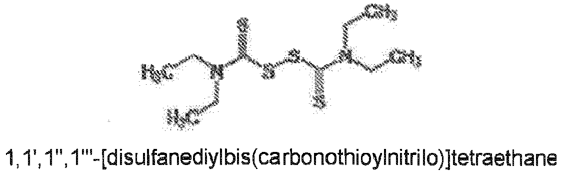
3. Epigallocatechin gallate (EGCG), including pharmaceutically acceptable salts thereof. Williamson et al., (December 2006), The Journal of Allergy and Clinical Immunology 118 (6): 1369-74.
   Epigallocatechin gallate has the following chemical structure and name. 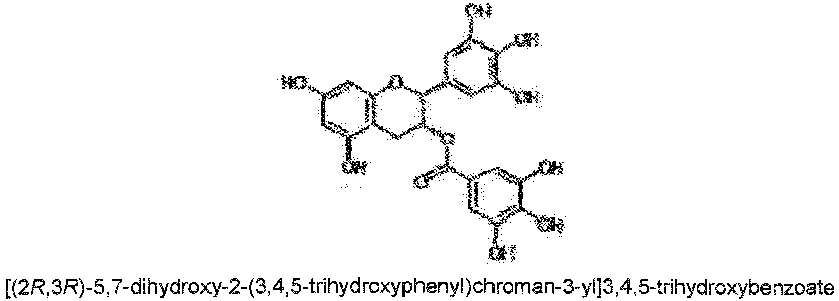
4. Salinosporamide A, including pharmaceutically acceptable salts thereof. Feling et at., (2003), Angew. Chem. Int. Ed. Engl. 42 (3): 355-7.
   Salinosporamide A has the following chemical structure and name. 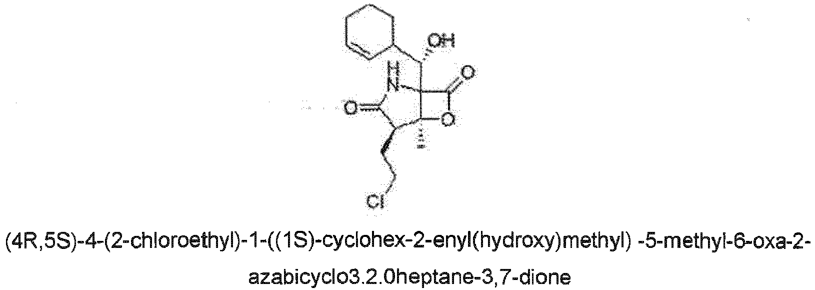
5. Carfilzomib, including pharmaceutically acceptable salts thereof. Kuhn DJ, et al, Blood, 2007, 110:3281-3290.
   Carfilzomib has the following chemical structure and name.

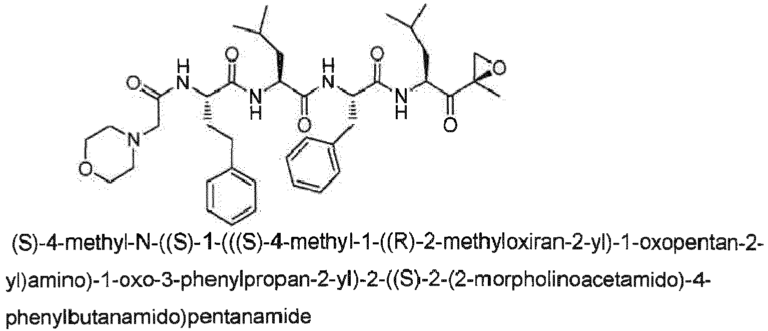

The 70 kilodalton heat shock proteins (Hsp70s) and 90 kilodalton heat shock proteins (Hsp90s) are a families of ubiquitously expressed heat shock proteins. Hsp70s and Hsp90s are over expressed certain cancer types. Several Hsp70s and Hsp90s inhibitors are being studied in the treatment of cancer. Suitable Hsp70s and Hsp90s inhibitors for use in combination herein include:
1. 17-AAG(Geldanamycin), including pharmaceutically acceptable salts thereof. Jia Wet al. Blood. 2003 Sep 1:102(5):1824-32.
   17-AAG(Geldanamycin) has the following chemical structure and name. 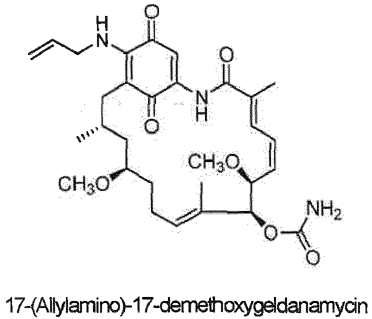
2. Radicicol, including pharmaceutically acceptable salts thereof. (Lee et al., Mol Cell Endocrinol. 2002, 188,47-54)
   Radicicol has the following chemical structure and name.

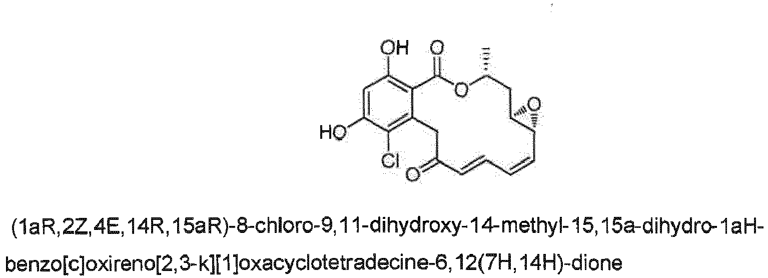

Inhibitors of cancer metabolism - Many tumor cells show a markedly different metabolism from that of normal tissues. For example, the rate of glycolysis, the metabolic process that converts glucose to pyruvate, is increased, and the pyruvate generated is reduced to lactate, rather than being further oxidized in the mitochondria via the tricarboxylic acid (TCA) cycle. This effect is often seen even under aerobic conditions and is known as the Warburg Effect.

Lactate dehydrogenase A (LDH-A), an isoform of lactate dehydrogenase expressed in muscle cells, plays a pivotal role in tumor cell metabolism by performing the reduction of pyruvate to lactate, which can then be exported out of the cell. The enzyme has been shown to be upregulated in many tumor types. The alteration of glucose metabolism described in the Warburg effect is critical for growth and proliferation of cancer cells and knocking down LDH-A using RNA-i has been shown to lead to a reduction in cell proliferation and tumor growth in xenograft models.
D. A. Tennant et. al., Nature Reviews, 2010, 267.
P. Leder, et. al., Cancer Cell, 2006, 9, 425.

High levels of fatty acid synthase (FAS) have been found in cancer precursor lesions. Pharmacological inhibition of FAS affects the expression of key oncogenes involved in both cancer development and maintenance.
Alli et al. Oncogene (2005) 24, 39-46. doi:10.1038

Inhibitors of cancer metabolism, including inhibitors of LDH-A and inhibitors of fatty acid biosynthesis (or FAS inhibitors), are suitable for use in combination with the compounds of this invention.

In one embodiment, the combination of the present invention comprises Compound A and Compound B and at least one anti-neoplastic agent selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine MEKngiogenesis inhibitors, immunotherapeutic agents, proapoptotic agents, and cell cycle signaling inhibitors.

In one embodiment, the combination of the present invention comprises Compound A and Compound B and at least one anti-neoplastic agent which is an anti-microtubule agent selected from diterpenoids and vinca alkaloids.

In a further embodiment, the at least one anti-neoplastic agent agent is a diterpenoid.

In a further embodiment, the at least one anti-neoplastic agent is a vinca alkaloid.

In one embodiment, the combination of the present invention comprises Compound A and Compound B and at least one anti-neoplastic agent, which is a platinum coordination complex.

In a further embodiment, the at least one anti-neoplastic agent is paclitaxel, carboplatin, or vinorelbine.

In a further embodiment, the at least one anti-neoplastic agent is carboplatin.

In a further embodiment, the at least one anti-neoplastic agent is vinorelbine.

In a further embodiment, the at least one anti-neoplastic agent is paclitaxel.

In one embodiment, the combination of the present invention comprises a compound of formula I and salts or solvates thereof and at least one anti-neoplastic agent which is a signal transduction pathway inhibitor.

In a further embodiment the signal transduction pathway inhibitor is an inhibitor of a growth factor receptor kinase VEGFR2, TIE2, PDGFR, BTK, erbB2, EGFr, IGFR-1, TrkA, TrkB, TrkC, or c-fms.

In a further embodiment the signal transduction pathway inhibitor is an inhibitor of a serine/threonine kinase rafk, akt, or PKC-zeta.

In a further embodiment the signal transduction pathway inhibitor is an inhibitor of a non- receptor tyrosine kinase selected from the src family of kinases.

In a further embodiment the signal transduction pathway inhibitor is an inhibitor of c-src.

In a further embodiment the signal transduction pathway inhibitor is an inhibitor of the androgen receptor.

In a further embodiment the signal transduction pathway inhibitor is an inhibitor of Ras oncogene selected from inhibitors of farnesyl transferase and geranylgeranyl transferase.

In a further embodiment the signal transduction pathway inhibitor is an inhibitor of a serine/threonine kinase selected from the group consisting of PI3K.

In a further embodiment the signal transduction pathway inhibitor is a dual EGFr/erbB2 inhibitor, for example N-{3-Chloro-4-[(3-fluorobenzyl) oxy]phenyl}-6-[5-({[2-(methanesulphonyl)ethyl]amino}methyl)-2-furyl]-4-quinazolinamine (structure below): 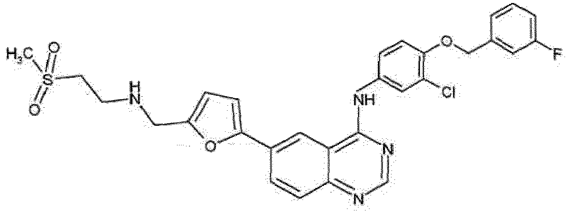 In one embodiment, the combination of the present invention comprises a compound of formula I or a salt or solvate thereof and at least one anti-neoplastic agent which is a cell cycle signaling inhibitor.

In further embodiment, cell cycle signaling inhibitor is an inhibitor of CDK2, CDK4 or CDK6.

In one embodiment the mammal in the methods and uses of the present invention is a human.

Suitably, the present invention relates to a method of treating or lessening the severity of a cancer that is either wild type or mutant for various genes or proteins representing individual components of each of the Raf, Ras, MEK, and/or PI3K/Pten signaling pathways. This includes but is not limited to patients having cancers that are mutant for RAF, wild type for RAS, wild type for MEK, and wild type for PI3K/PTEN; mutant for RAF, mutant for RAS, wild type for MEK, and wild type for PI3K/PTEN; mutant for RAF, mutant for RAS, mutant for MEK, and wild type for PI3K/PTEN; and mutant for RAF, wild type for RAS, mutant for MEK, and wild type PI3K/PTEN.

The term "wild type" as is understood in the art refers to a polypeptide or polynucleotide sequence that occurs in a native population without genetic modification. As is also understood in the art, a "mutant" includes a polypeptide or polynucleotide sequence having at least one modification to an amino acid or nucleic acid compared to the corresponding amino acid or nucleic acid found in a wild type polypeptide or polynucleotide, respectively. Included in the term mutant is Single Nucleotide Polymorphism (SNP) where a single base pair distinction exists in the sequence of a nucleic acid strand compared to the most prevalently found (wild type) nucleic acid strand.

Cancers that are either wild type or mutant for Raf, Ras, MEK, or mutant for PI3K/Pten are identified by known methods. For example, wild type or mutant tumor cells can be identified by DNA amplification and sequencing techniques, DNA and RNA detection techniques, including, but not limited to Northern and Southern blot, respectively, and/or various biochip and array technologies. Wild type and mutant polypeptides can be detected by a variety of techniques including, but not limited to immunodiagnostic techniques such as ELISA, Western blot or immunocyto chemistry. Suitably, Pyrophosphorolysis-activated polymerization (PAP) and/or PCR methods may be used. Liu, Q et al; Human Mutation 23:426-436 (2004).

The most common system for determining how far cancer has spread is the four-stage tumor/nodes/metastases system. Several different hormonal approaches are used in the management of various stages of prostate cancer including bilateral orchiectomy, estrogen therapy, luteinizing hormone-releasing hormone agonist therapy, antiandrogen therapy, androgen deprivation therapy and antiadrenal therapy. Radical prostatectomy is usually reserved for patients who are good health and elect surgical intervention and have tumor confined to the prostate gland (stage I and stage II). Patients who are considered poor medical candidates for radical prostatectomy and have confirmed pathologic diagnosis of stages I, II and III are candidates for radiation therapy.

According, the compounds of the present invention may be combined with prostate cancer treatment therapy including radical prostatectomy, radiation therapy, bilateral orchiectomy, estrogen therapy, luteinizing hormone-releasing hormone agonist therapy, antiandrogen therapy, androgen deprivation therapy and/or antiandrenal therapy.

Generally, the combinations of the present invention are tested for efficacy, advantageous and synergistic properties according to known procedures such as described below.

Suitably, the combinations of the invention are tested for efficacy, advantageous and synergistic properties generally according to the following combination cell proliferation assays. Cells are plated in 96 or 384-well plates at 500-5000 cells/well in culture media appropriate for each cell type, supplemented with 10% FBS and 1% penicillin/streptomycin, and incubated overnight at 37°C, 5% CO₂. Cells are treated in a grid manner with dilution of Compound A (8 dilutions, including no compound, of 3-fold dilutions starting from 0.1-30 µM depending on combination) from left to right on 96-well plate and also treated with Compound B (8 dilutions, including no compound, of 3-fold dilutions starting from 1-30 µM depending on combination) from top to bottom on 96-well plate and incubated as above for a further 72 hours. Optional additional antineoplastic agents may also be added. In some instances compounds are added in a staggered manner and incubation time can be extended up to 7days. Cell growth is measured using CellTiter-Glo® reagent according to the manufacturer's protocol and signals are read on a PerkinElmer EnVision™ reader set for luminescence mode with a 0.5-second read. Data are analyzed as described below.

Results are expressed as a percentage of control (untreated cells) and decrease in signal for each single agent at various concentrations is compared with the combination treatment at respective single agent concentrations. Alternatively, the results are expressed as a percentage of the t=0 value and plotted against compound(s) concentration. The t=0 value is normalized to 100% and represents the number of cells present at the time of compound addition. The cellular response is determined for each compound and/or compound combination using a 4- or 6-parameter curve fit of cell viability against concentration using the IDBS XLfit plug-in for Microsoft Excel software and determining the concentration required for 50% inhibition of cell growth (gIC₅₀). Background correction is made by subtraction of values from wells containing no cells. For each drug combination a Combination Index (CI), Excess Over Highest Single Agent (EOHSA) and Excess Over Bliss (EOBliss) are calculated according to known methods such as described in Chou and Talalay (1984) Advances in Enzyme Regulation, 22, 37 to 55; and Berenbaum, MC (1981) Adv. Cancer Research, 35, 269-335.

Because the combinations of the present invention are active in the above assays they exhibit advantageous therapeutic utility in treating cancer.

Suitably, the present invention relates to a method for treating or lessening the severity of prostate cancer.

This invention provides a combination comprising (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, and optional additional antineoplastic agents.

This invention also provides for a combination comprising (3β)-17-(pyridin-3-yl)androsta-5,16-dien-3-ol or a pharmaceutically acceptable salt or ester thereof, and *N-*{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof.

This invention also provides for a combination comprising (3β)-17-(pyridin-3-yl)androsta-5,16-dien-3-ol or a pharmaceutically acceptable salt or ester thereof, and *N-*{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, for use in treating cancer.

This invention also provides a pharmaceutical composition comprising a combination of (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof.

This invention also provides a combination kit comprising (3β)-17-(pyridin-3-yl)androsta-5,16-dien-3-ol or a pharmaceutically acceptable salt or ester thereof, and *N-*{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, and optional additional antineoplastic agents.

This invention also provides for the use of a combination comprising (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, in the manufacture of a medicament.

This invention also provides for the use of a combination comprising (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, in the manufacture of a medicament to treat cancer.

This invention also provides a method of treating cancer which comprises administering a combination of (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, and optional additional antineoplastic agents to a subject in need thereof.

Suitably, as used herein (3β)-17-(pyridin-3-yl)androsta-5,16-dien-3-ol or a pharmaceutically acceptable salt or ester thereof, is replaced by Compound A.

Suitably, as used herein *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof, is replaced by Compound B.

The following examples are intended for illustration only and are not intended to limit the scope of the invention in any way.

### Experimental Details

### Example 1 - Capsule Composition

An oral dosage form for administering a combination of the present invention is produced by filling a standard two piece hard gelatin capsule with the ingredients in the proportions shown in Table I, below.

**Table I**

| INGREDIENTS | AMOUNTS |
|---|---|
| (Compound A) | 250mg |
| (Compound B) | 72mg |
| Mannitol | 350 mg |
| Talc | 225 mg |
| Magnesium Stearate | 8 mg |

### Example 2 - Capsule Composition

An oral dosage form for administering one of the compounds of the present invention is produced by filing a standard two piece hard gelatin capsule with the ingredients in the proportions shown in Table II, below.

**Table II**

| INGREDIENTS | AMOUNTS |
|---|---|
| (Compound A) | 250mg |
| Mannitol | 300mg |
| Talc | 30mg |
| Magnesium Stearate | 4mg |

### Example 3 - Capsule Composition

An oral dosage form for administering one of the compounds of the present invention is produced by filing a standard two piece hard gelatin capsule with the ingredients in the proportions shown in Table III, below.

**Table III**

| INGREDIENTS | AMOUNTS |
|---|---|
| (Compound B) | 72mg |
| Mannitol | 150mg |
| Talc | 12mg |
| Magnesium Stearate | 8mg |

### Example 4 - Tablet Composition

The sucrose, microcrystalline cellulose and the compounds of the invented combination, as shown in Table IV below, are mixed and granulated in the proportions shown with a 10% gelatin solution. The wet granules are screened, dried, mixed with the starch, talc and stearic acid, then screened and compressed into a tablet.

**Table IV**

| INGREDIENTS | AMOUNTS |
|---|---|
| (Compound A) | 250mg |
| (Compound B) | 72mg |
| Microcrystalline cellulose | 400mg |
| sucrose | 40mg |
| starch | 20mg |
| talc | 10mg |
| stearic acid | 5mg |

### Example 5 - Tablet Composition

The sucrose, microcrystalline cellulose and one of the compounds of the invented combination, as shown in Table V below, are mixed and granulated in the proportions shown with a 10% gelatin solution. The wet granules are screened, dried, mixed with the starch, talc and stearic acid, then screened and compressed into a tablet.

**Table V**

| INGREDIENTS | AMOUNTS |
|---|---|
| (Compound A) | 250mg |
| Microcrystalline cellulose | 400mg |
| sucrose | 40mg |
| starch | 20mg |
| talc | 10mg |
| stearic acid | 2mg |

### Example 6 - Tablet Composition

The sucrose, microcrystalline cellulose and one of the compounds of the invented combination, as shown in Table VI below, are mixed and granulated in the proportions shown with a 10% gelatin solution. The wet granules are screened, dried, mixed with the starch, talc and stearic acid, then screened and compressed into a tablet.

**Table VI**

| INGREDIENTS | AMOUNTS |
|---|---|
| (Compound B) | 72mg |
| Microcrystalline cellulose | 300mg |
| sucrose | 40mg |
| starch | 20mg |
| talc | 10mg |
| stearic acid | 5mg |

### Example 7 - Injectable Parenteral Composition

An injectable form for administering a presently invented combinations is produced by stirring 1.5% by weight of (Compound A) and (Compound B) in 10% by volume propylene glycol in water.

### Example 8 - Injectable Parenteral Composition

An injectable form for administering a compound of the presently invented combinations is produced by stirring 1.5% by weight of (Compound A) in 10% by volume propylene glycol in water.

### Example 9 - Injectable Parenteral Composition

An injectable form for administering a compound of the presently invented combinations is produced by stirring 1.5% by weight of (Compound B) in 10% by volume propylene glycol in water.

While the preferred embodiments of the invention are illustrated by the above, it is to be understood that the invention is not limited to the precise instructions herein disclosed and that the right to all modifications coming within the scope of the following claims is reserved.

### EMBODIMENTS:

Although the present invention is defined in the attached claims, it should be understood that the present invention can also (alternatively) be defined in accordance with the following embodiments:
1. A combination comprising:
   (i) a compound of Structure (I) 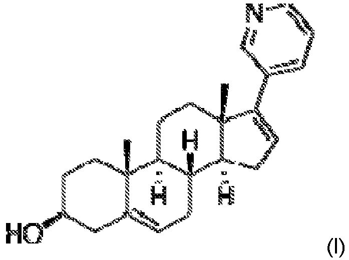 or a pharmaceutically acceptable salt or ester thereof;
      and
   (ii) a compound of Structure (II) 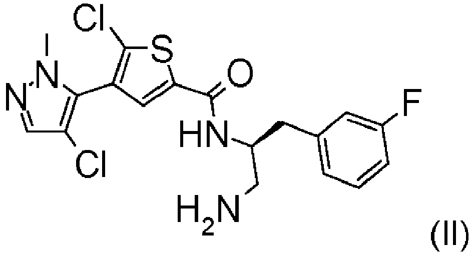 or a pharmaceutically acceptable salt thereof.
2. A combination according to embodiment 1, wherein compound (I) is in the form of an ester.
3. A combination according to embodiment 1, wherein compound (I) is in the form of an acetate ester.
4. A combination according to embodiment 1, wherein compound (II) is in the form of the hydrochloride salt.
5. A combination kit comprising a combination according embodiments 1 or 2 together with a pharmaceutically acceptable carrier or carriers.
6. Use of a combination according to any of embodiments 1 or 2 in the manufacture of a medicament for the treatment of cancer.
7. A combination according to embodiments 1 or 2 for use in therapy.
8. A combination according to embodiments 1 or 2 for use in treating cancer.
9. A pharmaceutical composition comprising a combination according to embodiments 1 to 2 together with a pharmaceutically acceptable diluent or carrier.
10. A method of treating cancer in a human in need thereof which comprises the administration of a therapeutically effective amount of
   (i) a compound of Structure (I) 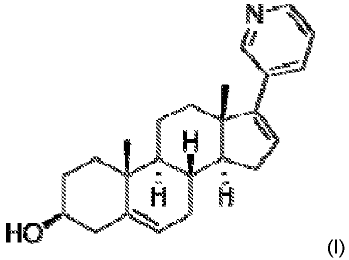 or a pharmaceutically acceptable salt or ester thereof;
      and
   (ii) a compound of Structure (II) 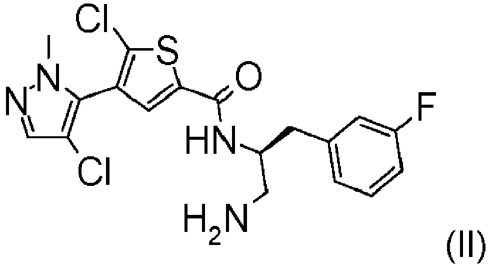 or a pharmaceutically acceptable salt thereof for use in therapy.
11. The method of embodiment 10, wherein the cancer is selected from head and neck cancer, breast cancer, lung cancer, colon cancer, ovarian cancer, prostate cancer, gliomas, glioblastoma, astrocytomas, glioblastoma multiforme, Bannayan-Zonana syndrome, Cowden disease, Lhermitte-Duclos disease, inflammatory breast cancer, Wilm's tumor, Ewing's sarcoma, Rhabdomyosarcoma, ependymoma, medulloblastoma, kidney cancer, liver cancer, melanoma, pancreatic cancer, sarcoma, osteosarcoma, giant cell tumor of bone, thyroid cancer, lymphoblastic T cell leukemia, Chronic myelogenous leukemia, Chronic lymphocytic leukemia, Hairy-cell leukemia, acute lymphoblastic leukemia, acute myelogenous leukemia, AML, Chronic neutrophilic leukemia, Acute lymphoblastic T cell leukemia, plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor), and testicular cancer.
12. The method of embodiments 10 and 11, wherein the cancer is prostate cancer.
13. The method of embodiments 10 and 11, wherein the cancer is a PTEN-deficient cancer.
14. The method of any one of embodiments 10 to 13, wherein compound (I) is in the form of the acetate ester and the compound (II) is in the form of the hydrochloride salt.
15. The method of treating cancer in a human in need thereof which comprises administering a therapeutically effective amount of a combination of (3β)-17-(pyridin-3-yl)androsta-5,16-dien-3-ol or a pharmaceutically acceptable salt or ester thereof, and *N-*{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof, to a human in need thereof, wherein the combination is administered within a specified period, and wherein the combination is administered for a duration of time.
16. The method of embodiment of 15, wherein (3β)-17-(pyridin-3-yl)androsta-5,16-dien-3-ol is in the form of the acetate ester.
17. The method of embodiment of 15, wherein N-{(1*S*)-2-amino-1-[(3-fluorophenyl) methyl]ethyl}-5-chloro-4-(4-chloro-1-methyl-1H-pyrazol-5-yl)-2- thiophenecarboxamide is in the form of the hydrochloride salt.
18. A combination according to embodiment 1 or embodiment 2 or of a combination kit according to embodiment 5 where the amount of the compound of Structure (I) is an amount selected from 750mg to 1,250mg, and that amount is suitable for administration once per day in one or more tablets, and the amount of the compound of Structure (II) is an amount selected from 50mg to 300mg, and that amount is suitable for administration once perday.
19. Use of a combination according to embodiment 1 or embodiment 2 or of a combination kit according to embodiment 5 in the manufacture of a medicament or medicaments for the treatment of cancer.
20. A combination or combination kit for use in the treatment of cancer, comprising a therapeutically effective amount of a combination of (3β)-17-(pyridin-3-yl)androsta-5,16-dien-3-ol or a pharmaceutically acceptable salt or ester thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof,
   wherein the combination is administered within a specified period, and
   wherein the combination is administered for a duration of time.
21. A combination or combination kit according to embodiment 20 wherein an amount of (3β)-17-(pyridin-3-yl)androsta-5,16-dien-3-ol acetate ester, is selected from about 750mg to about 1,250mg, and that amount is suitable for daily administration in one or more doses, and the amount of *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride, is selected from about 50mg to about 300mg, and that amount is suitable for administration once per day.
22. A combination according to embodiment 1 wherein (3β)-17-(pyridin-3-yl)androsta-5,16- dien-3-ol acetate ester and *N*-{(1*S*)-2-amino-1-[(3-fluorophenyl)methyl]ethyl}-5-chloro-4- (4-chloro-1-methyl-1*H*-pyrazol-5-yl)-2-thiophenecarboxamide hydrochloride, are administered within 12 hours of each other for from 1 to 3 consecutive days followed by administration of (3β)-17-(pyridin-3-yl)androsta-5,16-dien-3-ol acetate ester for from 3 to 7 consecutive days, optionally followed by one or more cycles of repeat dosing.
23. A combination or combination kit according to embodiment 21 wherein (3β)-17-(pyridin-3- yl)androsta-5,16-dien-3-ol acetate ester and *N*-{(1*S*)-2-amino-1-[(3-fluorophenyl) methyl]ethyl}-5-chloro-4-(4-chloro-1-methyl-1*H*-pyrazol-5-yl)-2-thiophenecarboxamide hydrochloride, are administered for at least 7 consecutive days.
24. A combination or combination kit according to embodiment 21 wherein (3β)-17-(pyridin-3- yl)androsta-5,16-dien-3-ol or a pharmaceutically acceptable salt or ester thereof, and *N*-{(1S)-2-amino-1-[(3-fluorophenyl)methyl]ethyl}-5-chloro-4-(4-chloro-1-methyl-1*H*-pyrazol-5-yl)-2-thiophenecarboxamide or a pharmaceutically acceptable salt thereof, are administered within 12 hours of each other for at least 5 consecutive days.
25. A combination or combination kit according to embodiment 24 wherein (3)-17-(pyridin-3- yl)androsta-5,16-dien-3-ol acetate ester and *N*-{(1S)-2-amino-1-[(3-fluorophenyl) methyl]ethyl}-5-chloro-4-(4-chloro-1-methyl-1H-pyrazol-5-yl)-2-thiophenecarboxamide hydrochloride, are administered for at least 14 consecutive days.
26. A combination or combination kit according to embodiment 1 or embodiment 5 wherein the compound (3)-17-(pyridin-3-yl)androsta-5,16-dien-3-ol acetate ester is first administered in a loading dose for from 1 to 3 days followed by maintenance dose administration of the compound, and/or the compound N-{(1S)-2-amino-1-[(3-fluorophenyl)methyl]ethyl}-5- chloro-4-(4-chloro-1-methyl-1H-pyrazol-5-yl)-2-thiophenecarboxamide hydrochloride is first administered in a loading dose for from 1 to 3 days followed by maintenance dose administration of the compound.
27. A combination comprising:
   (i) a compound of Structure (I) 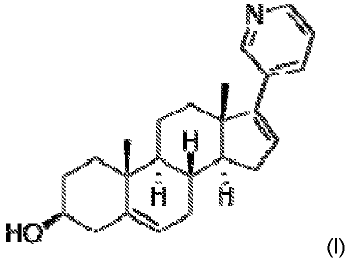 or a pharmaceutically acceptable salt or ester thereof;
      and
   (ii) a compound of Structure (X) 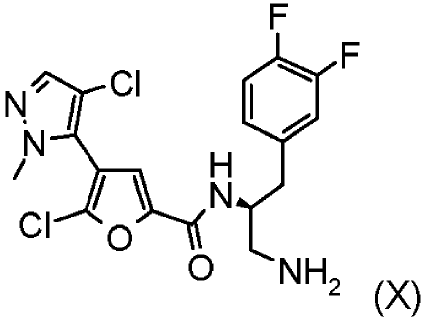 or a pharmaceutically acceptable salt thereof.
28. A combination according to embodiment 27, wherein compound (I) is in the form of an ester.
29. A combination according to embodiment 27, wherein compound (I) is in the form of an acetate ester.
30. A combination according to embodiment 27, wherein compound (X) is in the free of unsalted form.
31. A combination kit comprising a combination according embodiments 27 or 28 together with a pharmaceutically acceptable carrier or carriers.
32. Use of a combination according to any of embodiments 27 or 28 in the manufacture of a medicament for the treatment of cancer.
33. A combination according to embodiments 27 or 28 for use in therapy.
34. A combination according to embodiments 27 or 28 for use in treating cancer.
35. A pharmaceutical composition comprising a combination according to embodiments 27 or 28 together with a pharmaceutically acceptable diluent or carrier.
36. A method of treating cancer in a human in need thereof which comprises the administration of a therapeutically effective amount of
   (i) a compound of Structure (I) 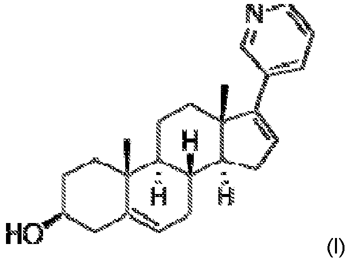 or a pharmaceutically acceptable salt or ester thereof;
      and
   (ii) a compound of Structure (X) 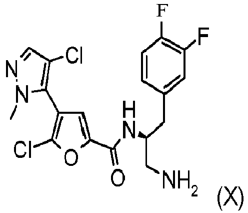 or a pharmaceutically acceptable salt thereof for use in therapy.
37. The method of embodiment 36, wherein the cancer is selected from head and neck cancer, breast cancer, lung cancer, colon cancer, ovarian cancer, prostate cancer, gliomas, glioblastoma, astrocytomas, glioblastoma multiforme, Bannayan-Zonana syndrome, Cowden disease, Lhermitte-Duclos disease, inflammatory breast cancer, Wilm's tumor, Ewing's sarcoma, Rhabdomyosarcoma, ependymoma, medulloblastoma, kidney cancer, liver cancer, melanoma, pancreatic cancer, sarcoma, osteosarcoma, giant cell tumor of bone, thyroid cancer, lymphoblastic T cell leukemia, Chronic myelogenous leukemia, Chronic lymphocytic leukemia, Hairy-cell leukemia, acute lymphoblastic leukemia, acute myelogenous leukemia, AML, Chronic neutrophilic leukemia, Acute lymphoblastic T cell leukemia, plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor), and testicular cancer.
38. The method of embodiments 36 or 37, wherein the cancer is prostate cancer.
39. The method of embodiments 36 or 37, wherein the cancer is a PTEN-deficient cancer.
40. The method of any one of embodiments 36 to 38, wherein compound (I) is in the form of the acetate ester and the compound (X) is in the free or unsalted form.
*41.* The method of treating cancer in a human in need thereof which comprises administering a therapeutically effective amount of a combination of (3β)-17-(pyridin-3-yl)androsta-5,16-dien-3-ol or a pharmaceutically acceptable salt or ester thereof, and *N-*{(1*S*)-2-amino-1-[(3,4-difluorophenyl)methyl]ethyl}-5-chloro-4-(4-chloro-1-methyl-1*H-*pyrazol-5-yl)-2-furancarboxamide, or a pharmaceutically acceptable salt thereof, to a human in need thereof, wherein the combination is administered within a specified period, and wherein the combination is administered for a duration of time.
42. The method of embodiment of 41, wherein (3β)-17-(pyridin-3-yl)androsta-5,16-dien-3-ol is in the form of the acetate ester.
43. The method of embodiment of 41, wherein *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide is in the free or unsalted form.
44. A combination according to embodiment 27 or embodiment 28 or of a combination kit according to embodiment 31 where the amount of the compound of Structure (I) is an amount selected from 750mg to 1,250mg, and that amount is suitable for administration once per day in one or more tablets, and the amount of the compound of Structure (X) is an amount selected from 50mg to 300mg, and that amount is suitable for administration once per day.
45. Use of a combination according to embodiment 27 or embodiment 28 or of a combination kit according to embodiment 31 in the manufacture of a medicament or medicaments for the treatment of cancer.
46. A combination or combination kit for use in the treatment of cancer, comprising a therapeutically effective amount of a combination of (3β)-17-(pyridin-3-yl)androsta-5,16-dien-3-ol or a pharmaceutically acceptable salt or ester thereof, and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, or a pharmaceutically acceptable salt thereof,
   wherein the combination is administered within a specified period, and
   wherein the combination is administered for a duration of time.
47. A combination or combination kit according to embodiment 46 wherein an amount of (3β)-17-(pyridin-3-yl)androsta-5,16-dien-3-ol acetate ester, is selected from about 750mg to about 1,250mg, and that amount is suitable for daily administration in one or more doses, and the amount of *N*-{(1*S*)-2-amino-1-[(3,4-difluorophenyl)methyl]ethyl}-5-chloro- 4-(4-chloro-1-methyl-1*H*-pyrazol-5-yl)-2-furancarboxamide, is selected from about 50mg to about 300mg, and that amount is suitable for administration once per day.
48. A combination according to embodiment 27 wherein (3β)-17-(pyridin-3-yl)androsta-5,16- dien-3-ol acetate ester and *N*-{(1*S*)-2-amino-1-[(3,4-difluorophenyl)methyl]ethyl}-5-chloro- 4-(4-chloro-1-methyl-1*H-*pyrazol-5-yl)-2-furancarboxamide, are administered within 12 hours of each other for from 1 to 3 consecutive days followed by administration of (3β)-17-(pyridin-3-yl)androsta-5,16-dien-3-ol acetate ester for from 3 to 7 consecutive days, optionally followed by one or more cycles of repeat dosing.
49. A combination or combination kit according to embodiment 47 wherein (3β)-17-(pyridin-3- yl)androsta-5,16-dien-3-ol acetate ester and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, are administered for at least 7 consecutive days.
*50.* A combination or combination kit according to embodiment 47 wherein (3β)-17-(pyridin-3- yl)androsta-5,16-dien-3-ol or a pharmaceutically acceptable salt or ester thereof, and N-{(1*S*)-2-amino-1-[(3,4-difluorophenyl)methyl]ethyl}-5-chloro-4-(4-chloro-1-methyl-1*H-*pyrazol-5-yl)-2-furancarboxamide or a pharmaceutically acceptable salt thereof, are administered within 12 hours of each other for at least 5 consecutive days.
51. A combination or combination kit according to embodiment 50 wherein (3β)-17-(pyridin-3- yl)androsta-5,16-dien-3-ol acetate ester and *N*-{(1*S*)-2-amino-1-[(3,4-difluorophenyl)methyl]ethyl}-5-chloro-4-(4-chloro-1-methyl-1*H*-pyrazol-5-yl)-2-furancarboxamide, are administered for at least 14 consecutive days.
52. A combination or combination kit according to embodiment 27 or embodiment 31 wherein the compound (3β)-17-(pyridin-3-yl)androsta-5,16-dien-3-ol acetate ester is first administered in a loading dose for from 1 to 3 days followed by maintenance dose administration of the compound, and/or the compound *N*-{(1*S*)-2-amino-1-[(3,4-difluorophenyl)methyl]ethyl}-5- chloro-4-(4-chloro-1-methyl-1*H*-pyrazol-5-yl)-2-furancarboxamide is first administered in a loading dose for from 1 to 3 days followed by maintenance dose administration of the compound.
53. A combination comprising:
   (i) an inhibitor of 17 a-hydroxylase/C17,20 lyase; and
   (ii) an inhibitor of AKT.

## Claims

1. A combination comprising:
(i) a first compound selected from CFG920 and abiraterone, or a pharmaceutically acceptable salt or ester thereof; and
(ii) a second compound of Structure (II) or Structure (X) 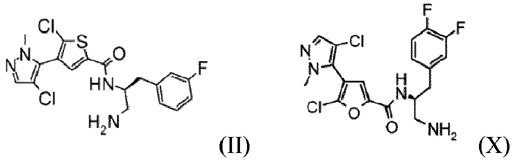 or a pharmaceutically acceptable salt thereof.

2. A combination according to claim 1, wherein the first compound is abiraterone in the form of an acetate ester.

3. A combination according to claim 1 or 2, wherein the second compound is the compound of Structure (II), optionally wherein the compound of Structure (II) is in the form of the hydrochloride salt.

4. A combination according to claim 1, wherein the second compound is the compound of Structure (X), optionally wherein the compound of Structure (X) is in the form of the free or unsalted form.

5. A combination according to claim 1, wherein the first compound is CFG920.

6. A combination according to claim 1, wherein the first compound is abiraterone, or a pharmaceutically acceptable salt or ester thereof, and the second compound is a compound of Structure (II), or a pharmaceutically acceptable salt thereof.

7. A combination according to claim 1, wherein the first compound is CFG920, and the second compound is a compound of Structure (II), or a pharmaceutically acceptable salt thereof.

8. A combination according to claim 1, wherein the first compound is abiraterone, or a pharmaceutically acceptable salt or ester thereof, and the second compound is a compound of Structure (X), or a pharmaceutically acceptable salt thereof.

9. A combination according to claim 1, wherein the first compound is CFG920, and the second compound is a compound of Structure (X), or a pharmaceutically acceptable salt thereof.

10. A combination kit comprising a combination according to any one of claims 1 to 9, together with a pharmaceutically acceptable carrier or carriers.

11. A pharmaceutical composition comprising a combination according to any one of claims 1 to 9, together with a pharmaceutically acceptable diluent or carrier.

12. A combination according to any one of claims 1 to 9 for use in a method of treating cancer.

13. A combination according to any one of claims 1 to 9 for use according to claim 12, wherein the cancer is selected from head and neck cancer, breast cancer, lung cancer, colon cancer, ovarian cancer, prostate cancer, gliomas, glioblastoma, astrocytomas, glioblastoma multiforme, Bannayan-Zonana syndrome, Cowden disease, Lhermitte-Duclos disease, inflammatory breast cancer, Wilm's tumor, Ewing's sarcoma, habdomyosarcoma, ependymoma, medulloblastoma, kidney cancer, liver cancer, melanoma, pancreatic cancer, sarcoma, osteosarcoma, giant cell tumor of bone, thyroid cancer, lymphoblastic T cell leukemia, Chronic myelogenous leukemia, Chronic lymphocytic leukemia, Hairy-cell leukemia, acute lymphoblastic leukemia, acute myelogenous leukemia, AML, Chronic neutrophilic leukemia, Acute lymphoblastic T cell leukemia, plasmacytoma, lmmunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor), and testicular cancer.

14. A combination according to any one of claims 1 to 9 for use according to claim 13, wherein the cancer is prostate cancer or wherein the cancer is a PTEN-deficient cancer.

15. A combination according to any one of claims 1 to 9, or a combination kit according to claim 10 where the amount of the first compound is an amount selected from 750mg to 1,250mg, and that amount is suitable for administration once per day in one or more tablets, and the amount of the second compound is an amount selected from 50mg to 300mg, and that amount is suitable for administration once per day.
